(19) Europäisches Patentamt
European Patent Office
Office européen des brevets

(11) EP 0 575 844 B1

(12) **EUROPEAN PATENT SPECIFICATION**

(45) Date of publication and mention of the grant of the patent:
**21.01.1998 Bulletin 1998/04**

(51) Int Cl.[6]: **C07C 259/06**, C07D 209/48, C07D 233/74, A61K 31/16, C07K 5/06

(21) Application number: **93109443.7**

(22) Date of filing: **14.06.1993**

(54) **Hydroxamic acid derivatives and pharmaceutical compositions thereof**

Hydroxamsäurederivate und deren pharmazeutische Zusammensetzungen

Composés d'acides hydroxamic et compositions pharmaceutiques de ceux-ci

(84) Designated Contracting States:
**AT BE CH DE DK ES FR GB GR IE IT LI LU MC NL PT SE**

(30) Priority: **25.06.1992 GB 9213473**
**05.04.1993 GB 9307081**

(43) Date of publication of application:
**29.12.1993 Bulletin 1993/52**

(73) Proprietor: **F. HOFFMANN-LA ROCHE AG**
**4002 Basel (CH)**

(72) Inventors:
- **Broadhurst, Michael John**
  **Barley, Royston, Herts. (GB)**
- **Brown, Paul Anthony**
  **Hitchin, Herts. (GB)**
- **Johnson, William Henry**
  **Hitchin, Herts. (GB)**
- **Lawton, Geoffrey**
  **Hitchin, Herts. (GB)**

(74) Representative: **Mahé, Jean et al**
**F.Hoffmann-La Roche AG**
**Patent Department (PLP),**
**124 Grenzacherstrasse**
**4070 Basel (CH)**

(56) References cited:
**EP-A- 0 214 639**
**EP-A- 0 231 081**
**EP-A- 0 236 872**
**EP-A- 0 489 577**
**WO-A-92/09556**

Remarks:
The file contains technical information submitted after the application was filed and not included in this specification

EP 0 575 844 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention is concerned with hydroxamic acid derivatives.

The hydroxamic acid derivatives provided by the present invention are compounds of the general formula

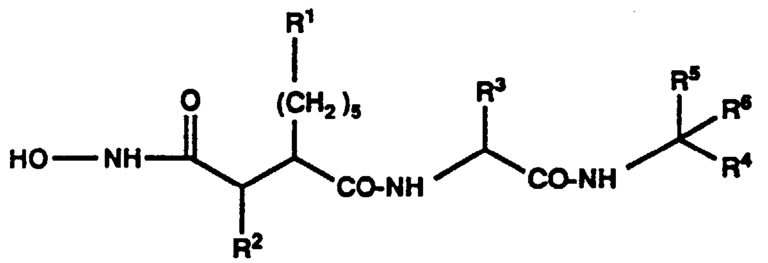

(I)

wherein

| | |
|---|---|
| $R^1$ | represents 2-7C straight-chain or branched-chain alkyl; |
| $R^2$ | represents hydrogen, 1-6C alkyl or a group of the formula $-(CH_2)_n$-aryl or $-(CH_2)_n$-Het in which n stands for 1-4 and Het represents a 5- or 6-membered N-heterocyclic ring which (a) is attached via the N atom, (b)optionally contains N, 0 and/or S as additional hetero atom(s) in a position or positions other than adjacent to the linking N atom, (c) is substituted by oxo on one or both C atoms adjacent to the linking N atom and (d) is optionally benz-fused or optionally substituted on one or more other carbon atoms by 1-6C alkyl or oxo and/or on any additional N atom(s) by 1-6C alkyl; |
| $R^3$ | represents the characterizing group of a natural or non-natural $\alpha$-amino acid in which any functional group present may be protected, with the proviso that $R^3$ does not represent hydrogen or bicycloaryl methylene; |
| $R^4$ | represents carboxyl, (1-6C alkoxy)carbonyl, carbamoyl or (1-6C alkyl)carbamoyl; |
| $R^5$ | represents the characterizing group of a naturally occurring $\alpha$-amino acid in which any functional group present may be protected; and |
| $R^6$ | represents hydrogen; or |
| $R^4$, $R^5$ and $R^6$ | each individually represent hydrogen or 1-6C alkyl, |

and pharmaceutically acceptable salts thereof.

The compounds of formula I possess valuable pharmacological properties. In particular, they are matrix metalloproteinase inhibitors and can be used in the control or prevention of degenerative joint diseases such as rheumatoid arthritis and osteoarthritis or in the treatment of invasive tumours, atherosclerosis or multiple sclerosis.

Matrix metalloprotease inhibitors have already been described in the art (WO-A-92/09566, EP-A- 231081, EP-A-214639, EP-A-489577) which are based on a similar peptide like structure as the compounds of the present invention. However, none of these references describe compounds with such type of alkyl chains in the $R^1$ position of the compounds of formula (I) of the present invention.

Objects of the present invention are the compounds of formula I and their pharmaceutically acceptable salts per se and for use as therapeutically active substances; a process for the manufacture of said compounds and salts; intermediates useful in said process; medicaments containing said compounds and salts and the manufacture of these medicaments; and the use of said compounds and salts in the control or prevention of illnesses or in the improvement of health, especially in the control or prevention of degenerative joint diseases or in the treatment of invasive tumours or atherosclerosis, or for the manufacture of a medicament for the control or prevention of degenerative joint diseases or for the treatment of invasive tumours, atherosclerosis or multiple sclerosis.

A 1-6C or 2-7C alkyl group alone or in combination, e.g. in alkoxy, preferably contains up to 4C atoms. It can be straight-chain or branched-chain, for example methyl, ethyl, n-propyl, isopropyl, n-butyl, isobutyl, sec.butyl, tert.butyl and the like. Methoxycarbonyl, ethoxycarbonyl, n-propoxycarbonyl, isopropoxycarbonyl and the like are examples of (1-6C alkoxy)carbonyl groups. A (1-6C alkyl)carbamoyl group can be, for example, methylcarbamoyl, ethylcarbamoyl, n-propylcarbamoyl, isopropylcarbamoyl and the like. The aryl moiety of a $-(CH_2)_n$-aryl group can be phenyl or naphthyl which is optionally substituted, for example by halogen, i.e. fluorine, chlorine, bromine or iodine, 1-6C alkyl, 1-6C alkoxy, trifluoromethyl and the like.

The characterizing group of a natural or non-natural $\alpha$-amino acid is the group R in a natural or non-natural $\alpha$-amino acid of the formula $H_2N-CH(R)-COOH$. Examples of such characterizing groups which are derived from natural

α-amino acids are the following, with the corresponding natural α-amino acid being indicated thereafter in parenthesis: hydrogen (glycine), methyl (alanine), isopropyl (valine), isobutyl (leucine), benzyl (phenylalanine), p-hydroxybenzyl (tyrosine), hydroxymethyl (serine), mercaptomethyl (cysteine), 1-hydroxyethyl (threonine), 2-methylthioethyl (methionine), carboxymethyl (aspartic acid), 2-carboxyethyl (glutamic acid) and 4-aminobutyl (lysine). Examples of such characterizing groups which are derived from non-natural α-amino acids are the following, with the corresponding non-natural α-amino acid being indicated thereafter in parenthesis: ethyl (α-amino-n-butyric acid), n-propyl (α-amino-n-pentanoic acid), n-butyl (α-amino-n-hexanoic acid), tert.butyl (α-amino-neohexanoic acid), neopentyl (α-amino-neoheptanoic acid), n-heptyl (α-amino-n-nonanoic acid), cyclohexylmethyl (cyclohexylalanine), phenyl (α-amino-phenylacetic acid), 2-phenyl-ethyl (homophenylalanine), 1-naphthyl (α-amino-1-naphthylacetic acid), 2-naphthyl (α-amino-2-naphthylacetic acid), phenethyl (α-amino-3-phenylbutyric acid), α-methylbenzyl (β-methylphenylalanine), α,α-dimethylbenzyl (β,β-dimethylphenylalanine) and the like.

Any functional (i.e. reactive) group present in $R^3$ and $R^5$ can be protected in a manner which is known per se in peptide chemistry. For example, an amino group can be protected by a tert.butoxycarbonyl, benzyloxycarbonyl, formyl, trityl, trifluoroacetyl, 2-(biphenylyl)isopropoxycarbonyl or isobornyloxycarbonyl group or in the form of a phthalimido group. A carboxy group can be protected, for example, in the form of a readily cleavable ester such as the methyl, ethyl, tert.butyl, benzyl or like ester. Protection of a hydroxy group can be in the form of an ether such as the methyl, tert.butyl, benzyl or tetrahydropyranyl ether or in the form of an ester such as the acetate. A mercapto group can be protected, for example, by a tert.butyl, benzyl or similar group.

The compounds of formula I form pharmaceutically acceptable salts with bases such as alkali metal hydroxides (e.g. sodium hydroxide and potassium hydroxide), alkaline earth metal hydroxides (e.g. calcium hydroxide and magnesium hydroxide), ammonium hydroxide and the like. The compounds of formula I which are basic form pharmaceutically acceptable salts with acids. As such salts there come into consideration not only salts with inorganic acids such as hydrohalic acids (e.g. hydrochloric acid and hydrobromic acid), sulphuric acid, nitric acid, phosphoric acid etc, but also salts with organic acids such as acetic acid, tartaric acid, succinic acid, fumaric acid, maleic acid, malic acid, salicylic acid, citric acid, methanesulphonic acid, p-toluenesulphonic acid etc.

The compounds of formula I contain at least two asymmetric carbon atoms and can accordingly exist as optically active enantiomers, as diastereoisomers or as racemates. The present invention is intended to embrace all of these forms.

In the compounds of formula I above, $R^1$ preferably represents ethyl, isopropyl n-butyl or n-hexyl, especially ethyl.

$R^2$ preferably represents hydrogen, methyl, a group of the formula $-(CH_2)_n$-aryl, wherein the aryl group is phenyl, or a group of the formula $-(CH_2)_n$-Het, wherein n has the significance given earlier and Het represents a 5- or 6-membered N-heterocyclic ring which optionally contains as additional hetero atom(s) one or two N atoms, one N atom and one O atom or one O atom. Het preferably represents a group of the formula

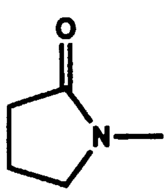

(a)

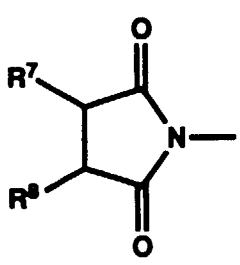

(b)

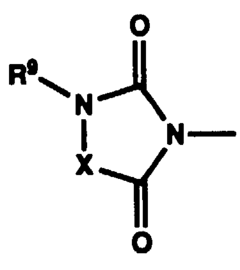

(c)

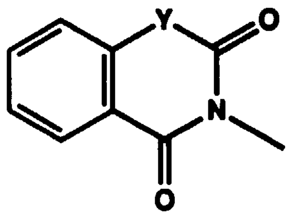

(d)

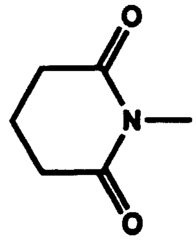

(e)

or

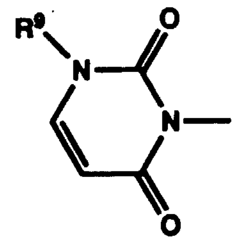

(f)

in which

| | |
|---|---|
| $R^7$ and $R^8$ | each represent hydrogen or together represent an additional bond or the remainder of a fused benzene ring; |
| $R^9$ | represents hydrogen or 1-6C alkyl; |
| X | represents -CO-, $-CH_2-$, -CH(1-6C alkyl)-, -C(1-6C alkyl)$_2$-, -NH-, -N(1-6C alkyl)- or -0-; and |
| Y | represents -O-, -NH- or -N(1-6C alkyl)-. |

In an especially preferred embodiment Het represents a group of formula (b), particularly phthalimido, or (c), particularly 3-methyl-2,5-dioxo-1-imidazolidinyl.

With respect to the symbol n, this preferably stands for 1 when $R^2$ represents a group of the formula $-(CH_2)_n$-Het and for 3 when $R^2$ represents a group of the formula $-(CH_2)_n$-aryl.

$R^3$ preferably represents methyl, isobutyl, tert.butyl, protected 4-aminobutyl, neopentyl, n-heptyl, cyclohexylmethyl, benzyl, α-methylbenzyl or α,α-dimethylbenzyl. 4-Phthalimidobutyl is the preferred protected 4-aminobutyl group.

Preferably, $R^4$ represents (1-6C alkyl)carbamoyl, especially methylcarbamoyl or ethylcarbamoyl, $R^5$ represents isobutyl and $R^6$ represents hydrogen or $R^4$, $R^5$ and $R^6$ each represent hydrogen, or $R^4$ and $R^5$ each represent hydrogen and $R^6$ represents tert.butyl or $R^4$, $R^5$ and $R^6$ each represent methyl.

Particularly preferred compounds of formula I above are:

[4-(N-Hydroxyamino)-2(R or S)-heptylsuccinyl]-L-leucyl-L-leucine ethylamide,
[4-(N-hydroxyamino)-2(R or S)-nonylsuccinyl]-L-leucyl-L-leucine ethylamide,
[4-(N-hydroxyamino)-2(R or S)-heptyl-3(S)-methylsuccinyl]-L-leucyl-L-leucine ethylamide,
[4-(N-hydroxyamino)-2(R)-heptyl-3(R or S)-(phthalimidomethyl)succinyl]-L-leucyl-L-leucine ethylamide,
[4-(N-hydroxyamino)-2(RS)-nonylsuccinyl]-L-tert-butylglycine methylamide,
[4-(N-hydroxyamino)-2(RS)-heptylsuccinyl]-L-phenylalanine methylamide,
[4-(N-hydroxyamino)-2(R)-heptyl-3(R or S)-(phthalimidomethyl)-succinyl]-L-tert.butylglycine methylamide and
[4-(N-hydroxyamino)-2(R)-heptyl-3(R or S)-(3-phenylpropyl)-succinyl]-L-leucyl-L-leucine ethylamide.

Examples of other interesting compounds of formula I above are:

[4-(N-Hydroxyamino)-2(RS)-heptylsuccinyl]-L-leucine methylamide,
[4-(N-hydroxyamino)-2(RS)-heptylsuccinyl]-L-leucine neopentylamide,
[4-(N-hydroxyamino)-2(RS)-heptylsuccinyl]-L-alanyl-L-leucine ethylamide,
[4-(N-hydroxyamino)-2(RS)-heptylsuccinyl]-L-($N^{\varepsilon}$-phthaloyl)-lysyl-L-leucine ethylamide,
[4-(N-hydroxyamino)-2(RS)-undecylsuccinyl]-L-leucyl-L-leucine ethylamide,
[4-(N-hydroxyamino)-2(RS)-heptylsuccinyl]-L-phenylalanyl-L-leucine ethylamide.
[4-(N-hydroxyamino)-2(RS)-heptylsuccinyl]-L-nonalyl-L-leucine ethylamide,
[4-(N-hydroxyamino)-2(RS)-heptylsuccinyl]-L-phenylalanine tert.butylamide,
[4-(N-hydroxyamino)-2(RS)-heptylsuccinyl]-L-tertbutylglycine methylamide,
[4-(N-hydroxyamino)-2(RS)-heptylsuccinyl]-L-neopentylglycine methylamide,
[4-(N-hydroxyamino)-2(RS)-heptylsuccinyl]-L-homophenylalanyl-L-leucine ethylamide,
[4-(N-hydroxyamino)-2(RS)-heptylsuccinyl]-L-cyclohexylalanine methylamide,
[4-(N-hydroxyamino)-2(RS)-isooctylsuccinyl]-L-phenylalanine methylamide,
[4-(N-hydroxyamino)-2(R)-heptylsuccinyl]-L-neopentylglycine methylamide,
[4-(N-hydroxyamino)-2(R)-heptylsuccinyl]-(D or L)-β,β-dimethylphenylalanine methylamide,

[4-(N-hydroxyamino) 2(R)-heptylsuccinyl]-(D or L)-threo-β-methylphenylalanine methylamide,
[4-(N-hydroxyamino) 2(R)-heptylsuccinyl]-DL-erythro-β-methylphenylalanine methylamide and
[4-(N-hydroxyamino)-2(R)-heptyl-3(R or S)-[(3-methyl-2,5-dioxo-1-imidazolidinyl)methyl]succinyl]-L-leucyl-L-leucine ethylamide.

According to the process provided by the present invention, the compounds of formula I hereinbefore and their pharmaceutically acceptable salts are manufactured by

(a) catalytically hydrogenating a compound of the general formula

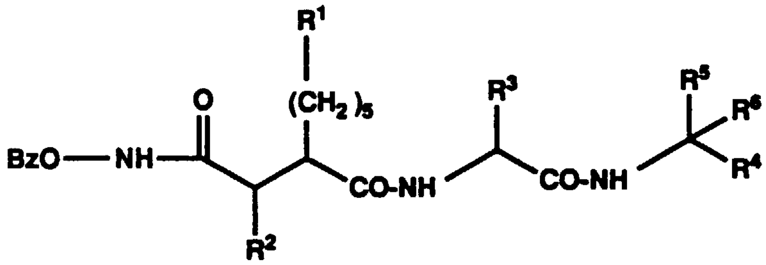

(II)

wherein $R^1$, $R^2$, $R^3$, $R^4$, $R^5$ and $R^6$ have the significance given earlier and Bz represents benzyl, or

(b) reacting an acid of the general formula

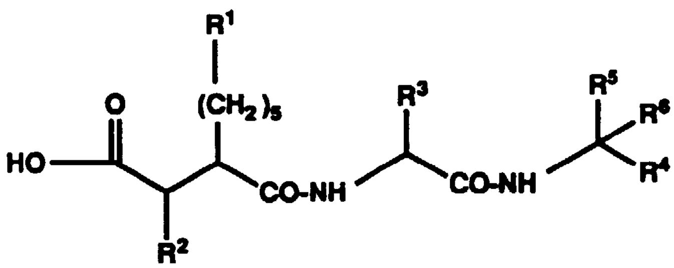

(III)

wherein $R^1$, $R^2$, $R^3$, $R^4$, $R^5$ and $R^6$ have the significance given earlier,

or an activated ester thereof with a compound of the general formula

$$H_2N\text{-}OZ \qquad (IV)$$

wherein Z represents hydrogen, tri(1-6C alkyl)silyl or diphenyl(1-6C alkyl)silyl, and, where required, cleaving off any diphenyl(lower alkyl)silyl group present in the reaction product, and,
if desired, converting a compound of formula I obtained into a pharmaceutically acceptable salt.

The catalytic hydrogenation of a compound of formula II in accordance with embodiment(a) of the process can be carried out in a manner known per se; for example in an inert organic solvent using hydrogen in the presence of a noble metal catalyst. Suitable inert organic solvents are, for example, 1-6C alkanols such as methanol, ethanol, etc. With respect to the catalyst, this can be, for example, a platinum, palladium or rhodium catalyst which can be supported on a suitable carrier material. Palladium/carbon is the preferred catalyst. The temperature and pressure are not critical, although for convenience the catalytic hydrogenation is preferably carried out at room temperature and under atmospheric pressure.

The reaction of an acid of formula III or an activated ester thereof with a compound of formula IV in accordance with embodiment (b) of the process can be carried out in a known manner. For example, when an acid is used, the reaction can be conveniently carried out in an inert organic solvent such as dimethylformamide or the like using hydroxy-benzotriazole in the presence of a condensation agent such as 1-ethyl-3-(3-dimethylaminopropyl)carbodiimide hydrochloride at about 0°C to about room temperature. When an activated ester of an acid of formula III is used, the reaction can be conveniently carried out in an inert organic solvent such as tetrahydrofuran at about 0°C to room temperature. Preferred compounds of formula IV are those in which Z represents hydrogen, tert.butyl-dimethylsilyl or tert.butyldiphenylsilyl. When a compound of formula IV in which Z represents tri(1-6C alkyl)silyl is used, this group is cleaved off during the reaction and working-up, and a compound of formula I is obtained directly. On the other hand, when a compound of formula IV in which Z represents diaryl-(1-6C alkyl)silyl is used, this group remains in the reaction product and must subsequently be cleaved off in a known manner, for example by means of fluoride ions.

Compounds of formula I can be converted into pharmaceutically acceptable salts by treatment with bases and basic compounds of formula I can be converted into pharmaceutically acceptable salts by treatment with acids. Such treatments can be carried out in a conventional manner.

The compounds of formulae II and III which are used as starting materials in the process are novel and are also an object of the present invention. They can be prepared, for example, by condensing a compound of the general formula

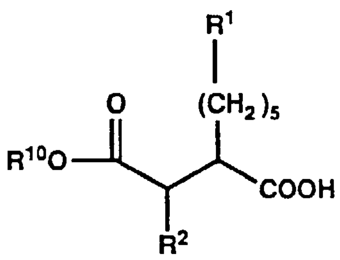

(V)

wherein $R^1$ and $R^2$ have the significance given earlier and $R^{10}$ represents a protecting group, with a compound of the general formula

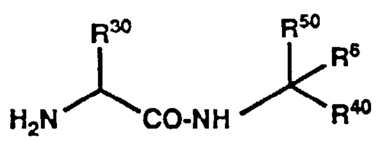

(VI)

wherein $R^6$ has the significance given earlier and $R^{30}$, $R^{40}$ and $R^{50}$ have the significances given earlier for $R^3$, $R^4$ and, respectively, $R^5$, but any functional group(s) present is/are protected, cleaving off the protecting group denoted by $R^{10}$ from the condensation product of the general formula

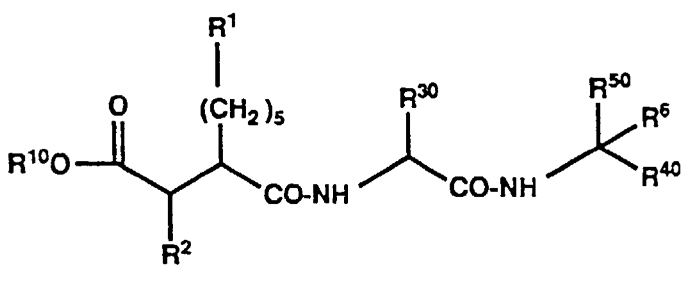

(VII)

wherein $R^2$, $R^6$, $R^{10}$, $R^{30}$, $R^{40}$ and $R^{50}$ have the significance given earlier, to give a compound of the general formula

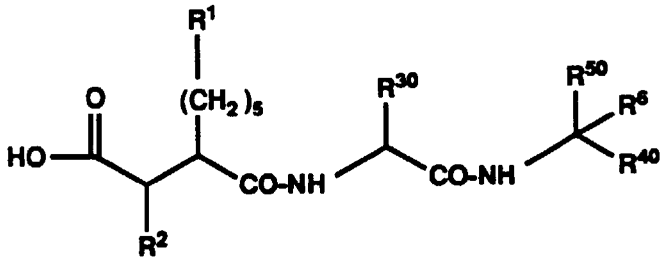

(IIIa)

wherein $R^1$, $R^2$, $R^6$, $R^{30}$, $R^{40}$ and $R^{50}$ have the significance given earlier,

and (i) where a compound of formula II is required, condensing a compound of formula IIIa, optionally before or after cleaving off protecting group(s) present in $R^{30}$, $R^{40}$ and $R^{50}$, with O-benzylhydroxylamine or (ii) where a compound of formula III is required in which functional group(s) present in $R^{30}$, $R^{40}$ and $R^{50}$ are not protected, cleaving off protecting group(s) present in a compound of formula IIIa.

The symbol denoted by $R^{10}$ in formula (V) can represent any conventional carboxy protecting group; for example methyl, ethyl, benzyl or, preferably, tert.butyl.

The condensation of a compound of formula V with a compound of formula VI can be carried out according to methods which are known per se in peptide chemistry; for example according to the acid halide, acid azide, acid anhydride, activated amide, mixed carbonic anhydride or activated ester method.

The cleavage of the protecting group $R^{10}$ from the condensation product of formula VII can be carried out in a manner known per se; for example by hydrolysis or, in the case of a tert.butyl protecting group, by treatment with anhydrous trifluoroacetic acid. It will be appreciated that protecting group(s) present in $R^{30}$, $R^{40}$ and $R^{50}$ may be concommitantly cleaved off depending on the nature of such groups.

The reaction of a compound of formula IIIa with O-benzylhydroxylamine can be carried out in a conventional manner; for example as described earlier in connection with the condensation of a compound of formula V with a compound of formula VI.

The cleavage of protecting groups present in $R^{30}$, $R^{40}$ and $R^{50}$ in the above description of the preparation of the starting materials of formulae II and III can be carried out according to methods known per se in peptide chemistry; for example by saponification, treatment with anhydrous trifluoroacetic acid or hydrogenolysis according to the nature of the protecting group.

The acids of formula III can be converted into the corresponding activated esters, which are also novel and form a further object of the present invention, in a manner known per se, for example by reaction with N-hydroxysuccinimide in the presence of dicyclohexylcarbodiimide to give the N-hydroxysuccinimide ester.

The compounds of formulae V and VI hereinbefore, insofar as they are not known compounds or analogues of known compounds, can be obtained as described in the Examples hereinafter or in analogy thereto.

As mentioned earlier, the hydroxamic acid derivatives provided by the present invention are matrix metalloproteinase inhibitors. This activity against stromelysin and gelatinase, two of such enzymes, can be demonstrated using the test procedures described hereinafter:

Stromelysin (Test A)

Prostromelysin was purified from human fibroblast culture medium by prostromelysin antibody affinity chromatography [Ganja-Smith Z, Nagase H, Woessner J F Jr., Biochem J. 1989, vol 285 (1), pp 115-119]. The latent pro-enzyme was activated by incubation with trypsin (5 mg/ml) at 25°C for 2 hours and after this time the trypsin was inactivated using a ten-fold excess of soya bean trypsin inhibitor. The inhibitory activity of the aforementioned hydroxamic acid derivatives was determined using $^{14}C$-acetylated b-casein substrate. The stromelysin (5 nM) was added to a solution of substrate (4 mg/ml) in 25 mM Tris HCl buffer, pH 7.5, containing 10 mM $CaCl_2$ and 0.05% Brij®. The solution obtained was incubated at 37°C for 20 hours. Enzyme digestion of the substrate was terminated by the addition of 45% trichloroacetic acid and the precipitated undigested substrate was pelleted by centrifugation at 10000 g for 0.25 hour. The supernatant was aspired and $^{14}C$ activity was determined by liquid scintillation spectrometry. The $IC_{50}$ is that concentration of hydroxamic acid derivative in the enzyme digestion which reduces the substrate cleavage to 50% of that achieved by the enzyme alone.

Stromelysin (Test B)

The latent pro-enzyme (30 µg/ml) was activated by incubation with trypsin (0.5 µg/ml) for 30 minutes at 25°C and

then the trypsin was inactivated using a twenty-fold excess of trypsin inhibitor. The inhibitory activity of the hydroxamic acid derivatives was determined using the fluorescent peptide substrate (7-methoxycoumarin-4-yl)acetyl-L-prolyl-L-leucyl-L-alanyl-L-nonalyl-[3-(2,4-dinitrophenyl)-L-2,3-diaminopropyl]-L-arginyl-L-arginine. The stromelysin (final concentration 35 ng/ml) was added to a solution of substrate (4 mM) and varying concentrations of hydroxylamine derivative in 50mM Tris HCl buffer, pH 7.5 containing 200 mM Na Cl, 10mM $CaCl_2$, 0.05% Brij 35 and 5% methanol. This solution was incubated for 16 hours at 37°C. Product formation was determined by spectrofluorometry using an excitation wavelength of 325 nm and an emission wavelength of 395 nm. The $IC_{50}$ is that concentration of hydroxylamine derivative which reduces the substrate cleavage to 50% of that achieved by the enzyme alone.

Gelatinase (Test C)

The in vitro inhibitor activity of the hydroxylamine derivatives against gelatinase B obtained from human neutrophils can be demonstrated using the following test procedure.

Pro-gelatinase B was purified from human neutrophils by first separating the neutrophils from human blood by density centrifugation and dextran sedimentation. The separated neutrophils were disrupted by combined treatment with 0.05% Triton X-100® and sonification Cell debris was then removed by high speed centrifugation. Pro-gelatinase B was purified from the supernatant by gelatine agarose affinity chromatography, the bound pro-enzyme being eluted from the affinity matrix by a 10% dimethyl sulphoxide/buffer wash. Analysis of the purified material revealed a single protein band with a molecular weight of 95 kDa when visualized by SDS-PAGE. The gelatinase B was incubated overnight at 37°C with 1mM p-aminophenylmercuric acetate. The inhibitory activity of the present hydroxamine derivatives was determined using the synthetic substrate N-acetyl-Pro-Gln-Gly-Leu-Leu-Gly ethyl ester for this enzyme. The gelatinase was added to a solution of 0.25 mM of substrate in 50mM borate buffer (pH 7.5) containing 1 mM calcium chloride and a known concentration of hydroxylamine derivative. The resulting solution was incubated at 37°C for 16 hours. Enzymic activity was terminated by addition of an equal volume of 2% (w/v) sodium hydrogen carbonate solution in 50% aqueous methanol containing 0.05% (w/v) of picrylsulphonic acid. The solution obtained was incubated at 37°C for a further 1 hour and the reaction was then terminated and stabilized by acidification with 1N hydrochloric acid. The concentration of trinitrophenyl-Leu-Leu-Gly ethyl ester formed was determined by spectrophotometry at 335 nm. The $IC_{50}$ is that concentration of hydroxylamine derivative which reduces substrate cleavage to 50% of that achieved by the enzyme alone.

The results obtained in the foregoing tests with representative hydroxamic acid derivatives provided by this invention are compiled in the following Table:

Table

| Compound of formula I | Test A $IC_{50}$ (mol) | Test B $IC_{50}$ (mol) | Test C $IC_{50}$ (mol) |
|---|---|---|---|
| A | $3.3 \times 10^{-8}$ | $1.25 \times 10^{-8}$ | $3.2 \times 10^{-7}$ |
| B | $2.6 \times 10^{-8}$ | $9.3 \times 10^{-9}$ | $5.0 \times 10^{-8}$ |
| C | $1.1 \times 10^{-8*}$ | $2.3 \times 10^{-9}$ | - |
| D | $1.0 \times 10^{-8*}$ | $5.9 \times 10^{-10*}$ | - |
| E | - | $1.8 \times 10^{-8}$ | $1.5 \times 10^{-9*}$ |
| F | - | $2.2 \times 10^{-8}$ | $3.2 \times 10^{-9*}$ |
| G | - | $1.2 \times 10^{-9}$ | $9.0 \times 10^{-10*}$ |
| H | | $6.6 \times 10^{-10*}$ | - |
| An asterisk * denotes that the $IC_{50}$ value was limited by mutual depletion of the enzyme. | | | |

Compound A: [4-(N-Hydroxyamino)-2(R or S)-heptylsuccinyl]-L-leucyl-L-leucine ethylamide.

Compound B: [4-(N-Hydroxyamino)-2(R or S)-nonylsuccinyl]-L-leucyl-L-leucine ethylamide.

Compound C: [4-(N-Hydroxyamino)-2(R or S)-heptyl-3(S)-methylsuccinyl]-L-leucyl-L-leucine ethylamide.

Compound D: [4-(N-Hydroxyamino)-2(R)-heptyl-3(R or S)-(phthalimidomethyl)succinyl]-L-leucyl-L-leucine ethylamide.

Compound E: [4-(N-Hydroxyamino-2(RS)-nonylsuccinyl]-L-tert.butylglycine methylamide.

Compound F: [4-(N-Hydroxyamino)-2(RS)-heptylsuccinyl]-L-phenylalanine methylamide.

Compound G: [4-(N-Hydroxyamino)-2(R)-heptyl-3(R or S)-(phthalimidomethyl)succinyl]-L-tert-butylglycine methylamide.

Compound H: [4-(N-Hydroxyamino) 2(R)-heptyl-3(R or S)-(3-phenylpropyl)succinyl]-L-leucyl-L-leucine ethylamide.

The compounds of formula I and their pharmaceutically acceptable salts can be used as medicaments, for example in the form of pharmaceutical preparations. The pharmaceutical preparations can be administered orally, e.g. in the form of tablets, coated tablets, dragées, hard and soft gelatine capsules, solutions, emulsions or suspensions. However, they can also be administered rectally, e.g. in the form of suppositories, or parenterally, e.g. in the form of injection solutions.

For the manufacture of pharmaceutical preparations the compounds of formula I and their pharmaceutically acceptable salts can be formulated with therapeutically inert, inorganic or organic carriers. Lactose, corn starch or derivatives thereof, talc, stearic acid or its salts can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active ingredient no carriers are, however, generally required in the case of soft gelatine capsules. Suitable carriers for the manufacture of solutions and syrups are, for example, water, polyols, saccharose, invert sugar, glucose and the like. Suitable carriers for the manufacture of injection solutions are, for example, water, alcohols, polyols, glycerine, vegetable oils and the like. Natural and hardened oils, waxes, fats, semi-liquid polyols and the like are suitable carriers for the manufacture of suppositories.

The pharmaceutical preparations can also contain preservatives, stabilizers, wetting agents, emulsifiers, sweeteners, colorants, flavorants, salts for adjustment of the osmotic pressure buffers coating agents or antioxidants.

Medicaments containing a compound of formula I or a pharmaceutically acceptable salt thereof and a therapeutically acceptable carrier as well as a process for the manufacture of such medicaments are also objects of the present invention. This process comprises mixing a compound of formula I or a pharmaceutically acceptable salt thereof with a therapeutically inert carrier material and bringing the mixture into a galenical administration form.

As mentioned earlier, the compounds of formula I and their pharmaceutically acceptable salts can be used in the control or prevention of illnesses, especially in the control or prevention of degenerative joint diseases or in the treatment of invasive tumours, atherosclerosis or multiple sclerosis. The dosage can vary within wide limits and will, of course, be adjusted to the individual requirements in each particular case. In general, in the case of administration to adults, a daily dosage of from about 5 mg to about 30 mg, preferably from about 10 mg to about 15 mg, should be appropriate, although the upper limit may be exceeded when this is found to be expedient. The daily dosage can be administered as a single dosage or in divided dosages.

The following Examples illustrate the present invention. The structure of the products was confirmed by NMR spectroscopy and mass spectroscopy. The composition of the thin-layer chromatography systems was as follows:

System A: chloroform:methanol:acetic acid:water 120:15:3:2;

System B: chloroform:methanol:acetic acid:water 240:24:3:2.

Further, all temperatures are given in degrees Centigrade.

Example 1

0.10 g (0.17 mmol) of [4-(N-benzyloxyamino)-2(R or S)-heptylsuccinyl]-L-leucyl-L-leucine ethylamide (isomer 1) in 10 ml of methanol was hydrogenated for 1 hour in the presence of 0.05 g of 5% palladium/ carbon. The mixture was filtered, the filtrate was evaporated and the residue was washed with diethyl ether and dried in vacuo to yield 0.083 g (98%) of [4-(N-hydroxyamino)-2(R or S)-heptylsuccinyl]-L-leucyl-L-leucine ethylamide as a hygroscopic white solid; Rf (System A) 0.54; MS: $(M+H)^+$ 485.

| Analysis for $C_{25}H_{48}N_4O_5$ 0.6$H_2O$: | | | |
|---|---|---|---|
| Calc. | C, 60.60; | H, 10.01; | N, 11.31% |
| Found | C, 60.62; | H, 9.75; | N, 11.39%. |

The [4-(N-benzyloxyamino)-2(R or S)-heptylsuccinyl]-L-leucyl-L-leucine ethylamide used as the starting material was prepared as follows:

(A) 25.95 g (0.15 mol) of DL-α-amino-nonanoic acid were dissolved in 900 ml of acetic acid while warming to 40°C. 20.7 g (0.3 mol) of sodium nitrite were added portionwise over 6 hours, with the heating being removed after about one third of the sodium nitrite had been added. The mixture was stirred for 16 hours at room temperature, evaporated, water was added and the oil obtained was extracted with diethyl ether (2 x 200 ml). The combined ether extracts were dried over anhydrous sodium sulphate and evaporated to give 31.9 g of a clear brown oil. This oil was dissolved in 150 ml methanol, a solution of 18 g of sodium hydroxide in 150 ml of water was added and the mixture was stirred at room temperature for 2 hours. The methanol was evaporated, water was added and the solution was acidified with 38 ml of concentrated hydrochloric acid . The product was extracted with diethyl ether (2 x 150 ml) and the extracts were dried over magnesium sulphate and evaporated to give a solid. Recrystallization from 120 ml of 60-80° petroleum ether yielded 20.58 g (79%) of 2(RS)-hydroxynonanoic acid of melting point 67-69°.

(B) 21.4 g (0.123 mol) of 2(RS)-hydroxynonanoic acid were dissolved in 240 ml of ethyl acetate. 17.3 ml (0.123 mol) of triethylamine and 14.8 ml (0.123 mol) of benzyl bromide were added and the mixture was heated under reflux for 3 hours. The mixture was cooled, filtered, washed with 2M hydrochloric acid, water and 5% sodium hydrogen carbonate solution and then dried over anhydrous magnesium sulphate. Evaporation of the solvent gave 25.4 g (78%) of benzyl 2(RS)-hydroxynonanoate as an oil; Rf (ethyl acetate/hexane 1:1) 0.68.

(C) A solution of 24.3 g (92 mmol) of benzyl 2(RS)-hydroxynonanoate and 8.9 ml (110 mmol) of pyridine in 150 ml of dry dichloromethane was added while stirring over a period of 0.5 hour to a solution of 18.6 ml (110 mmol) of trifluoromethanesulphonic anhydride in 200 ml of dry dichloromethane at 0°. The mixture was stirred at 0° for a further 1 hour. 3.7 ml (46 mmol) of pyridine and a solution of 7.7 ml (46 mmol) of trifluoromethanesulphonic anhydride in 100 ml of dry dichloromethane were added. The mixture was stirred for 1 hour at 0°, washed with water (2 x 100 ml), dried over magnesium sulphate and evaporated to give an oil. Chromatography on silica gel using ethyl acetate/hexane (1:9) for the elution yielded 26.7 g (73%) of benzyl 2(RS)-trifluoromethanesulphonyloxynonaoate; Rf (hexane/ethyl acetate 3:1) 0.66.

(D) A solution of 26.5 g (67 mmol) of benzyl 2(RS)-trifluoromethanesulphonyloxynonanoate in 100 ml of dry dichloromethane was added dropwise to a suspension of 2.11 g (70 mmol) of 80% sodium hydride and 16.75 g (67 mmol) of benzyl tert.butyl malonate in 170 ml of dry dimethylformamide at 0°. The mixture was stirred for 2 hours at 0° and for 16 hours at room temperature, diluted with dichloromethane, washed in succession with water, 5% sodium hydrogen carbonate solution and saturated sodium chloride solution, dried over magnesium sulphate and evaporated to yield an oil (34.6 g) containing dibenzyl 3-tert.butoxycarbonyl-2(RS)-heptylsuccinate; Rf (ethyl acetate/hexane 3:1) 0.49. The oil was dissolved in 540 ml of isopropanol and hydrogenated for 2 hours in the presence of 0.5 g of 5% palladium/carbon. The mixture was filtered, the filtrate was evaporated, toluene was added and the mixture was evaporated to remove traces of isopropanol. The residue (27.8 g) in 400 ml of toluene and 9.1 ml (65 mmol) of triethylamine was refluxed for 2 hours, evaporated and the residue was dissolved in ethyl acetate. The solution was washed with 0.5M hydrochloric acid (2 x 80 ml) and water, dried over magnesium sulphate and evaporated to yield 17.55 g (96%) of 4-tert.butyl hydrogen 2(RS)-heptylsuccinate; Rf (ethyl acetate) 0.63; MS: $(M+H)^+$ 273.

(E) 1.5 g (4 mmol) of N-tert.butoxycarhonyl-L-leucyl-L-leucine ethylamide were dissolved in 20 ml of 4M hydrogen chloride in ethyl acetate, stirred at room temperature for 0.75 hour, evaporated and the residue was dried in vacuo. The residue in 10 ml of dry dimethylformamide was treated at 0° in succession with 0.51 ml (4 mmol) of N-ethylmorpholine, a solution of 1.1 g (4 mmol) of 4-tert.butyl hydrogen 2(RS)-heptylsuccinate in 5 ml of dichloromethane, 0.82 g (6 mmol) of hydroxybenzotriazole and 0.92 g (4.4 mmol) of dicyclohexylcarbodiimide. The mixture was stirred for 1 hour at 0° and for 16 hours at room temperature. A few drops of 2-dimethylaminoethylamine were added, the suspension was cooled to 0° for 3 hours, filtered and evaporated. The residue was taken up in ethyl acetate, washed in succession with 5% citric acid solution, water, 5% sodium hydrogen carbonate solution and saturated sodium chloride solution, dried over magnesium sulphate and evaporated. Chromatography on silica gel using ethyl acetate/hexane (2:1) for the elution yielded 1.5 g (71%) of [4-tert.butoxy 2(RS)-heptyl-succinyl]-L-leucyl-L-leucine ethylamide as a white solid; Rf (dichloromethane/ methanol 19:1) 0.3; MS: $(M+H)^+$526.

(F) 0.8 g (1.5 mmol) of [4-tert.butoxy 2(RS)-heptylsuccinyl]-L-leucyl-L-leucine ethylamide was dissolved in 15 ml of trifluoroacetic acid and the solution was stirred for 2 hours and evaporated. The residue was evaporated three times with toluene, then dissolved in 10 ml of dry dichloromethane and cooled to 0°. 0.37 ml (4.5 mmol) of pyridine and 0.64 g (1.5 mmol) of di(1-benzotriazolyl)carbonate (70%) were added and the mixture was stirred for 1 hour at 0°. 0.22 ml (1.8 mmol) of O-benzylhydroxylamine was added and the mixture was stirred for 1 hour at 0° and

for 16 hours at room temperature. The resulting suspension was filtered, the solid was washed in succession with dichloromethane, 5% sodium hydrogen carbonate solution, water, 2M hydrochloric acid and water and then dried in vacuo. Chromatography on silica gel using methanol/dichloromethane (1:19) for the elution yielded 0.21 g (24%) of [4-(N-benzyloxyamino)-2(R or S)-heptylsuccinyl]-L-leucyl-L-leucine ethylamine (isomer 1); Rf (methanol/dichloromethane 1:19) 0.21; MS: $(M+H)^+$ 575; hplc (ODS reverse phase, eluted with 60% acetonitrile/ triethylammonium phosphate buffer pH 2.5) Rt 6.4 min.

The dichloromethane filtrate was washed in succession with 5% sodium hydrogen carbonate solution, 2M hydrochloric acid, 5% sodium hydrogen carbonate solution and saturated sodium chloride solution, dried over magnesium sulphate and evaporated. Chromatography on silica gel using methanol/dichloromethane (1:40) for the elution yielded 0.42 g (49%) of [4-(N-benzyloxyamino)-2(R or S)-heptylsuccinyl]-L-leucyl-L-leucine ethylamide as a mixture of isomers. Rf (methanol/dichloromethane 1:19) 0.20; MS: $(M+H)^+$ 575; hplc (ODS reverse phase, eluted with 60% acetonitrile/ triethylammonium phosphate buffer pH 2.5) Rt 6.4 and 11.3 mins.; ratio of isomer 1/isomer 2: 1:3.7

| Analysis for $C_{32}H_{54}N_4O_5$ (574.81) | | | |
|---|---|---|---|
| Calc. | C, 66.87; | H, 9.47; | N, 9.75% |
| Found | C, 66.51; | H, 9.41; | N, 9.68%. |

Example 2

0.5 g (1.12 mmol) of [4-(N-benzyloxyamino) 2(RS)-heptylsuccinyl]-L-leucine methylamide in 10 ml of methanol was hydrogenated for 2 hours in the presence of 0.01 g of 5% palladium/carbon. The mixture was filtered, the filtrate was evaporated and the residue was dried in vacuo to yield 0.36 g (90%) of [4-(N-hydroxyamino)-2(RS)-heptylsuccinyl]-L-leucine methylamide as a white powder; Rf (methanol/dichloromethane 1:19) 0.47 and 0.39; MS: $(M+H)^+$ 358.

| Analysis for $C_{18}H_{35}N_3O_4$ (357.50) | | | |
|---|---|---|---|
| Calc. | C, 60.48; | H, 9.87; | N, 11.75% |
| Found | C, 60.71; | H, 9.93; | N, 11.34%. |

The starting material was prepared as follows:

(A) 1.08 g of benzyloxycarbonyl-L-leucine methylamide in 20 ml of methanol containing 3.7 ml of 1M hydrochloric acid was hydrogenated for 2 hours in the presence of 0.05 g of 5% palladium/carbon. The mixture was filtered, the filtrate was evaporated and the residue was re-evaporated with toluene and then dried in vacuo. The residue was taken up in 10 ml of dimethylformamide, cooled to 0° and neutralized with 0.5 ml (3.9 mmol) of N-ethylmorpholine. A solution of 1.01 g (3.7 mmol) of 4-tert.butyl hydrogen 2(RS)-heptylsuccinate in 20 ml of dimethylformamide was added, followed by 0.6 g (4.4 mmol) of hydroxybenzotriazole and 0.84 g (4.08 mmol) of dicyclohexylcarbodiimide. The mixture was stirred for 1 hour at 0° and for 16 hours at room temperature and then evaporated. The residue was taken up in dichloromethane, filtered and the filtrate was washed in succession with two portions of 5% sodium hydrogen carbonate solution, water, 0.5M hydrochloric acid and water, dried over magnesium sulphate and evaporated to give an oil. Chromatography on silica gel using methanol/dichloromethane (3:97) for the elution yielded 0.99 g (67%) of [tert.butoxy 2(RS)-heptylsuccinyl]-L-leucine methylamide as an oil; Rf (ethyl acetate) 0.47 and 0.55; MS: $(M+H)^+$ 399.

(B) 0.96 g (2.4 mmol) of [tert.butoxy 2(RS)-heptylsuccinyl]-L-leucine methylamide was dissolved in 20 ml of trifluoroacetic acid, stirred at room temperature for 2 hours and evaporated. The residue was evaporated three times with toluene, then dissolved in 10 ml of dimethylformamide and cooled to 0°. The solution was neutralized by the addition of 0.61 ml (4.8 mmol) of N-ethylmorpholine. 0.39 g (2.89 mmol) of hydroxybenzotriazole and 0.57 g (2.97 mmol) of 1-ethyl-3-(3-dimethylaminopropyl)-carbodiimide hydrochloride were added and the solution was stirred for 1 hour at 0°. A solution of 0.35 g (2.8 mmol) of O-benzylhydroxylamine in 5 ml of dichloromethane was added, the mixture was stirred for 1 hour at 0° and overnight at room temperature and then evaporated. The oil was partitioned between 5% sodium hydrogen carbonate solution and dichloromethane. The organic layer was washed in succession with water, 2M hydrochloric acid and saturated sodium chloride solution, dried over magnesium sulphate and evaporated to give a solid. Chromatography on silica gel using methanol/hexane (1:9) for the elution yielded 0.56 g (52%) of [4-(N-benzyloxyamino)-2(RS)-heptyl-succinyl]-L-leucine methylamide as a white solid; Rf (ethyl acetate) 0.24 and 0.36; MS: $(M+H)^+$ 448.

Example 3

In a manner analogous to that described in the first paragraph of Example 2, from [4-(N-benzyloxyamino)-2(RS)-heptylsuccinyl]-L-leucine neopentylamide there was prepared [4-(N-hydroxyamino)-2(RS)-heptylsuccinyl]-L-leucine neopentylamide as a foam; Rf (methanol/dichloromethane 1:9) 0.52; MS: $(M+H)^+$ 414.

The starting material was prepared as follows:

From N-tert-butoxycarbonyl-L-leucine neopentylamide using methods analogous to those described in Example 1 (E-F) there was obtained [4-(N-benzyloxyamino)-2(RS)-heptylsuccinyl]-L-leucine neopentylamide as a white foam: Rf (methanol/dichloromethane 1:19) 0.59; MS $(M+H)^+$ 504.

Example 4

In a manner analogous to that described in the first paragraph of Example 2, from [4-(N-benzyloxyamino)-2(RS)-heptylsuccinyl]-L-alanyl-L-leucine ethylamide there was prepared [4-(N-hydroxyamino)-2(RS)-heptylsuccinyl]-L-alanyl-L-leucine ethylamide as a white solid; MS: $(M+H)^+$ 443.

| Analysis for $C_{22}H_{42}N_4O_5$ (442.60) | | | |
|---|---|---|---|
| Calc. | C, 59.70; | H, 9.57; | N, 12.66% |
| Found | C, 59.95; | H, 9.59; | N, 12.25%. |

The starting material was prepared as follows:

From N-benzyloxycarbonyl-L-alanyl-L-leucine ethylamide using methods analogous to those described in Example 2(A-B) there was obtained [4-(N-benzyloxyamino)-2(RS)-heptylsuccinyl]-L-alanyl-L-leucine ethylamide as a glassy solid; Rf (methanol/dichloromethane 1:19) 0.25 and 0.41; MS: $(M+H)^+$ 533.

Example 5

In a manner analogous to that described in the first paragraph of Example 2, from [4-(N-benzyloxyamino)-2(RS)-heptylsuccinyl]-L-($N^{\varepsilon}$-phthaloyl)-lysyl-L-leucine ethylamide there was prepared [4-(N-hydroxyamino)-2(RS)-heptylsuccinyl]-L-($N^{\varepsilon}$-phthaloyl)-lysyl-L-leucine ethylamide as a white solid; Rf (methanol/dichloromethane 1:19) 0.30; MS: $(M+H)^+$ 630

The starting material was prepared as follows:

From $N^{\alpha}$-benzyloxycarbonyl-$N^{\varepsilon}$-phthaloyl-L-lysyl-L-leucine ethylamide using methods analogous to those described in Example 2(A-B) there was obtained [4-(N-benzyloxyamino)-2(RS)-heptylsuccinyl]-L-($N^{\varepsilon}$-phthaloyl)-lysyl-L-leucine ethylamide as a foam; Rf(methanol/ dichloromethane 1:19) 0.41 and 0.50; MS: $(M+H)^+$ 720.

Example 6

0.2 g (0.33 mmol) of [4-(N-benzyloxyamino)-2(R or S)-nonylsuccinyl]-L-leucyl-L-leucine ethylamide (isomer 1) in 20 ml of methanol was hydrogenated for 80 minutes in the presence of 0.07 g of 5% palladium/carbon. The mixture was filtered, the filtrate was evaporated and the residue was dried in vacuo to yield 0.166 g (98%) of [4-(N-hydroxyamino)-2(R or S)-nonylsuccinyl]-L-leucyl-L-leucine ethylamide as a white solid; Rf(System B) 0.35; MS: $(M+H)^+$ 513.

| Analysis for $C_{27}H_{52}N_4O_5$ (512.74) | | | |
|---|---|---|---|
| Calc. | C, 63.25; | H, 10.22; | N, 10.93% |
| Found | C, 62.83; | H, 10.14; | N, 10.74% |

The starting material was prepared as follows:

(A) 6.25 ml (25 mmol) of dibenzyl malonate were dissolved in 25 ml of dry dimethylformamide under nitrogen and 0.75 g of 80% sodium hydride was added over a period of 10 minutes. The mixture was stirred for 0.5 hour, cooled to 0° and a solution of 4.75 ml (25 mmol) of 1-bromononane in 10 ml of dry dimethylformamide was added. Stirring at 0° was continued for 1 hour and the mixture was left at room temperature for 64 hours. The solution was evaporated and the residue was dissolved in diethyl ether. The organic phase was washed with water and saturated sodium chloride solution, dried over magnesium sulphate and evaporated. Chromatography on silica gel using ethyl acetate/ hexane (1:5) for the elution yielded 5.85 g (56%) of dibenzyl nonylmalonate as an oil; Rf (ethyl

acetate/hexane 1:3) 0.58.

(B) 5.85 g (14 mmol) of dibenzyl nonylmalonate were treated with 0.43 g (14 mmol) of 80% sodium hydride and 2.33 ml (14 mmol) of tert.butyl bromoacetate in the manner described in paragraph (A). Chromatography on silica gel using ethyl acetate/ hexane (1:19) for the elution yielded 6.24 g (83%) of benzyl 4-tert.butyl hydrogen (2-nonyl-2-benzyloxycarbonyl)succinate as an oil; Rf (ethyl acetate/hexane 1:19) 0.74.

(C) The benzyl 4-tert.butyl hydrogen (2-nonyl-2-benzyloxycarbonyl)succinate from paragraph B was dissolved in 80 ml of isopropanol and hydrogenated for 1 hour in the presence of 0.2 g of 5% palladium/carbon. The suspension was filtered, the filtrate was evaporated, 100 ml of toluene were added and the mixture was refluxed for 3 hours. Evaporation yielded 3.5 g (97%) of 4-tert.butyl hydrogen 2(RS)-nonylsuccinate as an oil; Rf (System A) 0.73.

(D) 1.5 g (4.0 mmol) of tert.butoxycarbonyl-L-leucyl-L-leucine -L-leucine ethylamide were dissolved in 20 ml of 4M hydrochloric acid in ethyl acetate, stirred at room temperature for 0.5 hour, evaporated, washed with diethyl ether and dried in vacuo. The solid was taken up in 10 ml of dry dimethylformamide, treated with 0.6 ml (4.7 mmol) of N-ethylmorpholine, 1.2 g (4.0 mmol) of 4-tert.butyl hydrogen 2(RS)-nonylsuccinate, 0.66 g (4.9 mmol) of hydroxybenzotriazole and 0.78 g (4.0 mmol) of 1-ethyl-3-(3-dimethylaminopropyl)carbodiimide hydrochloride. The mixture was stirred at 0° for 1 hour, left to stand for 16 hours at room temperature and evaporated. The residue was taken up in ethyl acetate, washed in succession with 5% sodium hydrogen carbonate solution, water, 5% citric acid solution water, 5% sodium hydrogen carbonate solution and saturated sodium chloride solution and then evaporated to give a solid. Chromatography on silica gel using ethyl acetate for the elution yielded 1.9 g (86%) of [4 tert. butoxy 2(RS)-nonylsuccinyl]-L-leucyl-L-leucine ethylamide as a white solid; Rf (ethyl acetate) 0.60; MS: $(M+H)^+$ 554.

(E) 0.95 g (1.7 mmol) of [4 tert.butoxy 2(RS)-nonylsuccinyl]-L-leucyl-L-leucine ethylamide was dissolved in 15 ml of trifluoroacetic acid and the solution was stirred for 2 hours at room temperature and evaporated. The residue was evaporated with toluene, dissolved in dichloromethane, washed with water, dried over magnesium sulphate and evaporated. The residue was taken up in 10 ml of dimethylformamide at -15° and treated with 0.22 ml (1.7 mmol) of N-ethylmorpholine and 0.22 ml (1.7 mmol) of isobutyl chloroformate. After 10 minutes at -15° 0.42 ml (3.4 mmol) of O-benzylhydroxylamine was added and the mixture was stirred for 1 hour at 0° and left to stand for 16 hours at room temperature. The mixture was diluted with water, the solid which separated was filtered off, washed in succession with 5% citric acid solution, water and 5% sodium hydrogen carbonate solution and then dried in vacuo. Chromatography of the residue on silica gel using methanol/ dichloromethane (3:97) for the elution yielded 0.55 g (54%) of [4-(N-benzyloxyamino)-2(RS)-nonylsuccinyl]-L-leucyl-L-leucine ethylamide as a white solid; Rf (methanol/dichloromethane 1:19) 0.47; MS: $(M+H)^+$ 603; hplc (ODS reverse phase, eluted with 70% acetonitrile/ trimethylammonium phosphate buffer pH 2.5) Rt 4.7 and 7.7 mins.

The isomers were separated as follows:

1.0 g (1.66 mmol) of the mixture was dissolved in 10 ml of hot dichloromethane and the solution was left to stand at room temperature for 2 hours and at 4° for 16 hours. The solid was filtered off, washed with dichloromethane and dried in vacuo to yield 0.41 g (41%) of [4-(N-benzyloxyamino)-2(R or S)-nonylsuccinyl]-L-leucyl-L-leucine ethylamide (isomer 1) as a solid; Rf(System B) 0.46; hplc Rt 4.6 mins.

The mother liquors were evaporated to yield 0.47 g (47%) of [4-(N-benzyloxyamino)-2(R or S)-nonylsuccinyl]-L-leucyl-L-leucine ethylamide (isomer 2) as a solid; Rf (System B) 0.52; hplc Rt 7.5 mins.

Example 7

In a manner analogous to that described in the first paragraph of Example 6, from [4-(N-benzyloxyamino)-2(RS)-nonylsuccinyl]-L-tert.butylglycine methylamide there was prepared [4-(N-hydroxyamino)-2(RS)-nonylsuccinyl]-L-tert-butylglycine methylamide as a white foam; Rf (System A) 0.58 and 0.64; MS: $(M+H)^+$ 386.

| Analysis for $C_{20}H_{39}N_3O_4.0.4\ CH_3OH$ | | | |
|---|---|---|---|
| Calc. | C, 61.51; | H, 10.27; | N, 10.55% |
| Found | C, 61.50; | H, 10.13; | N, 10.27%. |

The starting material was prepared as follows:

In a manner analogous to that described in Example 6 (D-E), but starting with L-tert.butylglycine methylamide in place of the deprotected tert.butoxycarbonyl-L-leucyl-L-leucine ethylamide, there was obtained [4-(N-benzyloxyamino)-2(RS)-nonylsuccinyl]-L-tert.butylglycine methylamide as a foam; Rf (methanol/dichloromethane 1:19) 0.27 and 0.34; MS(EI): $M^+$ 475.

Example 8

In a manner analogous to that described in the first paragraph of Example 6, from [4-(N-benzyloxyamino)-2(RS)-undecylsuccinyl]-L-leucyl-L-leucine ethylamide there was prepared [4-(N-hydroxyamino)-2(RS)-undecylsuccinyl]-L-leucyl-L-leucine ethylamide as a foam; Rf (System A) 0.62 and 0.66; MS: $(M+H)^+$ 541.

The starting material was prepared as follows:

In a manner analogous to that described in Example 6 (A-E), but using 1-bromoundecane in place of 1-bromononane, there was obtained [4-(N-benzyloxyamino)-2(RS)-undecylsuccinyl]-L-leucyl-L-leucine ethylamide as a gel; Rf (methanol/dichloromethane 1:9) 0.70; MS: $(M+H)^+$ 631.

Example 9

0.3 g (0.536 mmol) of [4-(N-benzyloxyamino)-2(R or S)-heptyl-3(S)-methylsuccinyl]-L-leucyl-L-leucine ethylamide (diastereoisomer 1) in 50 ml of methanol was hydrogenated for 1.5 hours in the presence of 0.1 g of 5% palladium/carbon. The mixture was filtered, the filtrate was evaporated and the residue was triturated with diethyl ether to yield 0.23 g of [4-(N-hydroxyamino)-2(R or S)-heptyl-3(S)-methylsuccinyl]-L-leucyl-L-leucine ethylamide (diastereoisomer 1) as a white solid; MS: 499 $(M+H)^+$.

| Analysis for $C_{26}H_{50}N_4O_5$ 0.4$H_2O$ | | | |
|---|---|---|---|
| Calc. | C 61.72; | H 10.12; | N 11.08 |
| Found | C 61.92; | H 10.05; | N 10.85 |

The starting material was prepared as follows:

(A) 2.0 g (0.11 mol) of L-lactic acid benzyl ester and 10.8 ml (0.13 mol) of pyridine in 220 ml of dichloromethane were added over a period of 0.5 hour to a solution of trifluoromethanesulphonic anhydride in 250 ml of dichloromethane at 0°. After a further 0.5 hour the solution was washed twice with 250 ml of water each time and with 250 ml of saturated sodium chloride solution and then dried over anhydrous magnesium sulphate. The solution was then added over 0.5 hours to a solution of di-tert.butyl sodio malonate (0.11 mol), (prepared from 22.8 g of di-tert-butyl malonate and 3.48 g of 80% sodium hydride in oil) in 250 ml of dimethylformamide at 0°. The mixture was left to warm to room temperature overnight and the solvent was evaporated. The residue was partitioned between 200 ml of ethyl acetate and 200 ml of 5% sodium bicarbonate solution. The organic layer was washed with water (2 x 200 ml), dried over anhydrous magnesium sulphate and evaporated to give a yellow oil. Flash chromatography on silica gel using 10% ethyl acetate in hexane for the elution gave 35.4 g (88%) of 1-benzyl 4-tert.butyl 3(RS)-tert.butoxycarbonyl-2(S)-methylsuccinate as a colourless oil; Rf (30% ethyl acetate in hexane) 0.63.

(B) 952 mg of 80% sodium hydride were added to a stirred solution of 10 g of 1-benzyl 4-tert.butyl 3(RS)-tert. butoxy-carbonyl-2(S)-methylsuccinate in 100 ml of dry dimethylformamide. The solution was stirred at room temperature for 2 hours and then 6.23 ml of 1-bromoheptane were added. The mixture was heated at 60°C for 4 hours and then stirred at room temperature overnight. The solvent was evaporated and the residue was dissolved in ethyl acetate. The solution was washed in succession with 5% sodium bicarbonate solution, water and saturated sodium chloride solution and then dried over anhydrous magnesium sulphate. The solvent was evaporated and the residue was purified by flash chromatography using 5% ethyl acetate in hexane for the elution to give 6.83 g of benzyl 3,3(RS)-di-tert.butoxycarbonyl-2(S)-methyldecanoate as a colourless oil; Rf (10% ethyl acetate in hexane) 0.5.

(C) 6.8 g of benzyl-3,3(RS)-di-tertbutoxycarbonyl-2(S)-methyldecanoate in 20 ml of trifluoroacetic acid were stirred at room temperature for 1 hour. The solvent was evaporated and the residue was re-evaporated from toluene (2 x 25 ml) to leave an orange oil. The oil was re-dissolved in 100 ml of toluene, treated with 3.64 ml of N-ethylmorpholine and heated under reflux for 1 hour. The solution was washed in succession with 2M hydrochloric acid,

water and sodium chloride solution, dried over anhydrous magnesium sulphate and evaporated to leave a brown oil. Purification by flash chromatography using 2% methanol in dichloromethane for the elution gave 3.02 g of 1-benzyl-3(RS)-carboxy-2(S)-methyldecanoate as a yellow oil; Rf (5% methanol in dichloromethane) 0.47,0.52; MS: 321(M+H)+.

(D) In a manner analogous to that described in Example 1, paragraphs (D)-(F), from 1.5 g of benzyl 3(RS)-carboxy-2(S)-methyldecanoate there was obtained 0.315 g of [4-(N-benzyloxyamino)-2(R or S)-heptyl-3(S)-methylsuccinyl]-L-leucyl-L-leucine ethylamide (diastereoisomer 1) as an off-white solid.

Example 10

0.25 g of [4-(N-benzyloxyamino)-2(R)-heptyl-3(R or S)-(phthalimidomethyl)succinyl]-L-leucyl-L-leucine ethylamide (diastereomer 1) in 100 ml of dimethylformamide was hydrogenated in the presence of 100 mg of 10% palladium/ carbon for 24 hours. The catalyst was filtered off and the solvent was evaporated. Trituration of the residue with diethyl ether gave 0.17 g of [4-(N-hydroxyamino)-2(R)-heptyl-3(R or S)-(phthalimidomethyl)succinyl]-L-leucyl-L-leucine ethylamide (diastereomer 1) as a grey powder; Rf (10% methanol in dichloromethane) 0.5; MS: 644 (M+H)+.

| Analysis for $C_{34}H_{53}N_5O_7$ 0.8$H_2O$ | | | |
|---|---|---|---|
| Calc. | C 62.04; | H 8.36; | N 10.64 |
| Found | C 61.99; | H 8.04; | N 10.60. |

The starting material was prepared as follows:

(A) In a manner analogous to that described in Example 1, paragraphs (A)-(D), from 20 g of D-α-amino-nonanoic acid there was obtained 10.28 g of dibenzyl 3(RS)-tert.butoxycarbonyl-2(R)-heptylsuccinate as a pale yellow oil; Rf(10% ethyl acetate in hexane) 0.32.

(B) 8.2 g of dibenzyl 3(RS)-tert.butoxycarbonyl-2(R)-heptylsuccinate in 100 ml of dry dimethylformamide were treated with 0.49 g of 80% sodium hydride dispersion After 2 hours the mixture was cooled to 0°C and 4.18 g of N-bromomethylphthalimide were added portionwise. The mixture was stirred at 0°C for a further 1 hour and then at room temperature overnight. The solvent was evaporated and the residue was partitioned between ethyl acetate and 5% sodium bicarbonate solution. The organic phase was washed with water and saturated sodium chloride solution and then dried over anhydrous magnesium sulphate. The solvent was evaporated and the residue purified by flash chromatography on silica gel using 20% ethyl acetate in hexane for the elution to give 6.42 g of dibenzyl 3(RS)-tert.butoxycarbonyl-2(R)-heptyl-3(RS)-(phthalimidomethyl)succinate.

(C) 3.5 g of dibenzyl 3(RS)-tertbutoxycarbonyl-2(R)-heptyl-3(RS)-(phthalimidomethyl)succinate in 50 ml of ethanol was hydrogenated in the presence of 0.35 g of 5% palladium/carbon. The catalyst was filtered off and the solvent was evaporated. The residue was dissolved in 50 ml of toluene, treated with 0.58 ml of N-ethylmorpholine and heated under reflux for 2 hours. The solution was washed in succession with 2M hydrochloric acid, water and saturated sodium chloride solution and then dried over anhydrous magnesium sulphate. Purification of the residue by flash chromatography on silica gel using 4% methanol in dichloromethane for the elution gave 1.54 g of 4-tert. butyl hydrogen 2(R)-heptyl-3(RS)-(phthamidomethyl)succinate as an oil; Rf(5% methanol in dichloromethane) 0.3.

(D) In a manner analogous to Example 1, paragraphs (E)-(F), from 0.6 g of 4-tert.butyl 2(R)-heptyl-3(RS)-(phthalimidomethyl)succinate there was obtained 0.27 g of [4-(N-benzyloxyamino)-2(R)-heptyl-3(R or S)-(phthalimidomethyl)succinyl]-L-leucyl-L-leucine ethylamide (diastereomer 1) as a white solid; MS: 734 (M+H)+.

| Analysis for $C_{41}H_{59}N_5O_7$ 0.6$H_2O$ | | | |
|---|---|---|---|
| Calc. | C 66.12 | H 8.15; | N 9.4 |
| Found | C 66.08; | H 8.08; | N 9.53. |

Example 11

In a manner analogous to that described in the first paragraph of Example 2, from [4-(N-benzyloxyamino)2(RS)-heptylsuccinyl]-L-phenylalanyl-L-leucine ethylamide there was prepared [4-(N-hydroxyamino)-2(RS)-heptylsuccinyl]-L-phenylalanyl-L-leucine ethylamide as a white solid; Rf (methanol/dichloromethane 1:19) 0.28 and 0.43; MS: $(M+H)^+$ 519.

| Analysis for $C_{28}H_{46}N_4O_5$. 0.3 $H_2O$ | | | |
|---|---|---|---|
| Calc. | C, 64.17; | H, 8.96; | N, 10.69% |
| Found | C, 64.07; | H, 8.77; | N, 10.64%. |

The starting material was prepared as follows:

From N-tert.butyloxycarbonyl-L-phenylalanyl-L-leucine ethylamide using methods analogous to those described in Example 1 (E-F) there was obtained [4-(N-benzyloxyamino)-2(RS)-heptylsuccinyl]-L-phenylalanyl-L-leucine ethylamide as a white solid; Rf (methanol/ dichloromethane 1:19) 0.64; MS: $(M+H)^+$ 609.

Example 12

In a manner analogous to that described in the first paragraph of Example 2, from [4-(N-benzyloxyamino)-2(RS)-heptylsuccinyl]-L-nonalyl-L-leucine ethylamide there was prepared [4-(N-hydroxyamino)-2(RS)-heptylsuccinyl]-L-nonalyl-L-leucine ethylamide as a white solid; MS: $(M+H)^+$ 527.

| Analysis for $C_{28}H_{54}N_4O_5$ (526.76) | | | |
|---|---|---|---|
| Calc. | C, 63.84; | H, 10.33; | N, 10.64% |
| Found | C, 63.64; | H, 10.39; | N, 10.63%. |

The starting material was prepared as follows:

From N-tert.butoxycarbonyl-L-nonalyl-L-leucine ethylamide using methods analogous to those described in Example 1 (E-F) there was obtained [4-(benzyloxyamino)-2(RS)-heptylsuccinyl]-L-nonalyl-L-leucine ethylamide as a white solid; Rf (methanol/dichloromethane 1:19) 0.55 and 0.62; MS: $(M+H)^+$ 617.

Example 13

In a manner analogous to that described in the first paragraph of Example 2, from [4-(N-benzyloxyamino)-2(RS)-heptylsuccinyl]-L-phenylalanine tert.butylamide there was prepared [4-(N-hydroxyamino)-2(RS)-heptylsuccinyl]-L-phenylalanine tert.butylamide as a hydroscopic foam; Rf (methanol/chloroform 1:19) 0.31; MS $(M+H)^+$ 434.

The starting material was prepared as follows:

From N-tert.butoxycarbonyl-L-phenylalanine tert.butylamide using methods analogous to those described in Example 1 (E-F) there was obtained [4-(N-benzyloxyamino)-2(RS)-heptylsuccinyl]-L-phenylalanine tert.butylamide as a white solid; Rf(ethyl acetate) 0.75; MS: $(M+H)^+$ 524.

Example 14

In a manner analogous to that described in the first paragraph of Example 2, from [4-(N-benzyloxyamino)-2(RS)-heptylsuccinyl]-L-phenylalanine methylamide there was prepared [4-(N-hydroxyamino)2(RS)-heptylsuccinyl]-L-phenylalanine methylamide as a white solid; Rf (methanol/dichloromethane 1:19) 0.16 and 0.21; MS: $(M+H)^+$ 392.

The starting material was prepared as follows:

From N-tert.butoxycarbonyl-L-phenylalanine methylamide using methods analogous to those described in Example 1 (E-F) there was obtained [4-(N-benzyloxyamino)-2(RS)-heptylsuccinyl]-L-phenylalanine methylamide as a white solid; Rf (methanol/chloroform 1:19) 0.29 and 0.36.

| Analysis for $C_{28}H_{39}N_3O_4$ (481.64) | | | |
|---|---|---|---|
| Calc. | C, 69.83; | H, 8.16; | N, 8.72% |
| Found | C, 69.56; | H, 8.05; | N, 9.00%. |

Example 15

In a manner analogous to that described in the first paragraph of Example 2, from [4-(N-benzyloxyamino)-2(RS)-heptylsuccinyl]-L-tert.butylglycine methylamide there was prepared [4-(N-hydroxyamino)2(RS)-heptylsuccinyl]-L-tert. butylglycine methylamide as a foam; Rf (methanol/dichloromethane 1:19) 0.23 and 0.29; MS; $(M+H)^+$ 358.

| Analysis for $C_{18}H_{35}N_3O_4$. 0.3 $CH_3OH$. | | | |
|---|---|---|---|
| Calc. | C, 59.87; | H, 9.94; | N, 11.45% |
| Found | C, 59.80; | H, 9.76; | N, 11.44%. |

The starting material was prepared as follows:

In a manner analogous to that described in Example 1 (E-F), but starting with L-tert.butylglycine methylamide in place of the deprotected benzyloxycarbonyl-L-leucine methylamide, there was prepared [4-(N-benzyloxyamino)-2(RS)-heptylsuccinyl]-L-tert.butylglycine methylamide as a white foam; Rf (methanol/chloroform 1:19) 0.29 and 0.36; MS: $(M+H)^+$ 448.

Example 16

In a manner analogous to that described in the first paragraph of Example 2, from [4-(N-benzyloxyamino)-2(RS)-heptylsuccinyl]-L-neopentylglycine methylamide there was prepared [4-(N-hydroxyamino)-2(RS)-heptylsuccinyl]-L-neopentylglycine methylamide as a white solid; MS: $(M+H)^+$ 372.

| Analysis for $C_{13}H_{37}N_3O_4$ (371.52) | | | |
|---|---|---|---|
| Calc. | C, 61.43; | H, 10.04; | N, 11.31% |
| Found | C, 61.04; | H, 09.93; | N, 11.13%. |

The starting material was prepared as follows:

From N-tert.butoxycarbonyl-L-neopentylglycine methylamide using methods analogous to those described in Example 1 (E-F) there was obtained [4-(N-benzyloxyamino)-2(RS)-heptylsuccinyl]-L-neopentylglycine methylamide as a white foam; Rf (methanol/chloroform 1:19) 0.47 and 0.55; MS: $(M+H)^+$ 462.

| Analysis for $C_{26}H_{43}N_3O_4$ (461.65) | | | |
|---|---|---|---|
| Calc. | C, 67.65; | H, 9.39; | N, 9.10% |
| Found | C, 67.57; | H, 9.12; | N, 9.02%. |

Example 17

In a manner analogous to that described in the first paragraph of Example 2, from [4-(N-benzyloxyamino)-2(RS)-heptylsuccinyl]-L-homophenylalanyl-L-leucine ethylamide there was prepared [4-(N-hydroxyamino)-2(RS)-heptylsuccinyl]-L-homophenylalanyl-L-leucine as a white hygroscopic granular powder; MS: $(M+H)^+$ 533.

| Analysis for $C_{29}H_{48}N_4O_5$.0.4$H_2O$ | | | |
|---|---|---|---|
| Calc. | C, 64.51; | H, 9.11; | N, 10.38% |
| Found | C, 64.59; | H, 8.94; | N, 10.14%. |

The starting material was prepared as follows:

From N-benzyloxyoarbonyl-L-homophenylalanyl-L-leucine ethylamide using methods analogous to these described in Example 2 (A-B) there was obtained [4-(N-benzyloxyamino)-2(RS)-heptylsuccinyl]-L-homophenylalanine-L-leucine ethylamide as a solid; Rf (methanol/chloroform 1:19) 0.55; MS: $(M+H)^+$ 623.

| Analysis for $C_{36}H_{54}N_4O_5$ (622.85) | | | |
|---|---|---|---|
| Calc. | C, 69.42; | H, 8.74; | N, 9.00% |
| Found | C, 69.02; | H, 8.85; | N, 8.87%. |

Example 18

In a manner analogous to that described in the first paragraph of Example 2, from [4-(N-benzyloxyamino)-2(RS)-heptylsuccinyl]-L-cyclohexylalanine methylamide there was prepared [4-(N-hydroxyamino)-2(RS)-L-cyclohexylalanine methylamide as a white solid; MS: $(M+H)^+$ 398.

| Analysis for $C_{21}H_{39}N_3O_4$ (397.56). | | | |
|---|---|---|---|
| Calc. | C, 63.45; | H, 09.89; | N, 10.57% |
| Found | C, 63.24; | H, 10.04; | N, 10.33%. |

The starting material was prepared as follows:

From N-tert.butoxycarbonyl-L-cyclohexylalanine methylamide using methods analogous to those described in Example 1 (E-F) there was prepared [4-(N-benzyloxyamino)-2(RS)-heptylsuccinyl]-L-cyclohexylalanine methylamide as a white solid; Rf (methanol/dichloromethane 1:9) 0.30 and 0.37; MS: $(M+H)^+$ 488.

Example 19

0.6 g (1.14 mmol) of [4-tert.butoxy 2(RS)-heptylsuccinyl]-L-leucyl-L-leucine ethylamide [prepared in Example 1 (E)] was dissolved in 10 ml of trifluoroacetic acid and the solution was stirred for 3 hours and evaporated. The residue was evaporated four times from toluene, then dissolved in 200 ml of dichloromethane containing 5 ml of methanol, washed twice with water, dried over magnesium sulphate and evaporated. The residue was dissolved in 1 ml of dimethylformamide and 10 ml of 1,2-dimethoxyethane at 0°. 0.135 g (1.17 mmol) of N-hydroxysuccinimide and 0.243 g (1.17 mmol) of dicyclohexylcarbodiimide were added, the mixture was stirred for 1 hour at 0°, and for 16 hours at 4°, filtered and evaporated to yield the crude N-hydroxysuccinimide ester as a sticky solid. This ester was suspended in 5 ml of tetrahydrofuran, treated with a solution prepared from 0.178 g (2.56 mmol) of hydroxylamine hydrochloride and 0.094 g (2.35 mmol) of sodium hydroxide in 5 ml of water, stirred for 16 hours at room temperature, filtered and evaporated. The resulting gum was washed with water by decantation and dried by evaporation of toluene. Chromatography on silica gel using 1% of methanol in dichloromethane and then 2%, 3% and 5% methanol in dichloromethane for the elution and evaporation gave [4-(N-hydroxyamino)-2(R or S)-heptylsuccinyl]-L-leucyl-L-leucine ethylamide, isomers 1 and 2, as white solids. Rf (methanol/dichloromethane 1:12:5) 0.34 (isomer 2) and Rf 0.26 (isomer 1).

Isomer 1 was washed with dichloromethane and ethyl acetate and then dried to yield 0.083 g (15%) of [4-(N-hydroxyamino)-2(R or S)-heptylsuccinyl]-L-leucyl-L-leucine ethylamide (isomer 1) identical to that prepared in Example 1. Rf (system A) 0.54. MS: $(M+H)^+$ 485.

| Analysis for $C_{25}H_{48}N_4O_5$ $0.2H_2O$ | | | |
|---|---|---|---|
| Calc. | C, 61.50; | H, 9.99; | N, 11.47% |
| Found | C, 61.41; | H, 10.09; | N, 11.48%. |

Example 20

0.30 g (0.6 mmol) of [4-(N-benzyloxyamino)-2(RS)-isooctylsuccinyl]-L-phenylalanine methylamide in 200 ml of methanol was hydrogenated for 3 hours in the presence of 0.1 g of 10% palladium/carbon. The mixture was filtered and the filtrate was evaporated to yield 0.20 g (81%) of [4-(N-hydroxyamino)-2(RS)-isooctylsuccinyl]-L-phenylalanine methylamide as a white solid; MS: $(M+H)^+$ 406.

| Analysis for $C_{22}H_{35}N_3O_4$ (405.54) | | | |
|---|---|---|---|
| Calc. | C, 65.16; | H, 8.70; | N, 10.36% |
| Found | C, 65.04; | H, 8.75; | N, 10.20%. |

The [4-(N-benzyloxyamino)-2(RS)-isooctylsuccinyl]-L-phenylalanine methylamide used as the starting material was prepared as follows:

(A) A solution of 13.03 g (60 mmol) of diethyl acetamidomalonate in 50 ml of dry dimethylformamide was added over 0.5 hour to a suspension of 2.16 g (72 mmol) of 80% sodium hydride in 50 ml of dry dimethylformamide at 0° under a nitrogen atmosphere. The mixture was stirred for 3 hours at room temperature, a solution of 15.05 g

(78 mmol) of 1-bromo-6-methylheptane in 50 ml dry dimethylformamide was added and the mixture stirred for 64 hours and evaporated to an oil, The oil was extracted twice with ethyl acetate, the combined extracts were washed with water, dried over magnesium sulphate and evaporated. The residue was refluxed in 6M hydrochloric acid (180 ml) for 4 hours, cooled and the waxy solid was filtered off. The crude product in 120 ml of 50% aqueous ethanol was filtered, 7 ml of pyridine were added and the mixture was cooled in ice to yield 6.49 g (57%) of DL-isooctylglycine as a fine off-white powder.

(B) 6.45 g (34.5 mmol) of DL-isooctylglycine were suspended in 200 ml of acetic acid at room temperature. 4.8 g (69 mmol) of sodium nitrite were added portionwise over 3 hours. The clear solution was stirred for 16 hours at room temperature, evaporated, water and diethyl ether were added and the mixture was filtered. The ether solution was dried over magnesium sulphate and evaporated to an oil. The recovered and dried starting material (1.76 g) was recycled according to the above procedure to yield an ether-soluble oil. The combined product (7.43 g) was dissolved in 16 ml methanol, a solution of 3.9 g of sodium hydroxide in 33 ml of water was added and the mixture was stirred at room temperature for 2 hours. The methanol was evaporated, water was added and the solution was extracted with diethyl ether and acidified with concentrated hydrochloric acid. The product was extracted with diethyl ether and, after, evaporation, yielded 5.88 g (91%) of 2(RS)-hydroxyisooctanoic acid as a clear oil which solidified on standing.

(C) 5.84 g (31.3 mmol) of 2(RS)-hydroxyisooctanoic acid were dissolved in 60 ml of ethyl acetate. 4.4 ml (31.3 mmol) of triethylamine and 3.8 ml (31.3 mmol) of benzyl bromide were added and the mixture was heated under reflux for 3 hours. The mixture was cooled, filtered, washed in succession with 2M hydrochloric acid, water and 5% sodium hydrogen carbonate solution, dried over magnesium sulphate and evaporated. Chromatography on silica gel using ethyl acetate/hexane (3:7), for the elution yielded 5.55 g (64%) of benzyl 2(RS)-hydroxyisooctenoate as an oil Rf (ethyl acetate/hexane 1:1) 0.50.

(D) A solution of 5.45 g (19.6 mmol) of benzyl 2(RS)-hydroxyisooctanoate and 1.91 ml (23.5 mmol) of pyridine in 35 ml of dry dichloromethane was added while stirring over a period of 0.5 hour to a solution of 4 ml (23.5 mmol) of trifluoromethanesulphonic anhydride in 40 ml of dry dichloromethane at 0°. The mixture was stirred for 1 hour at 0° and for 1 hour at room temperature. The solution was washed twice with water, dried over magnesium sulphate and evaporated to give 7.32 g (95%) of benzyl 2(RS)-trifluoromethanesulphonyloxyisooctanoate; Rf (ethyl acetate/hexane 1:4) 0.73

(E) A solution of 7.31 g (18.55 mmol) of benzyl 2(RS)-trifluoromethanesulphonyloxyisooctanoate in 30 ml of dry dichloromethane was added dropwise over 1 hour to a suspension of 0.58 g (19.5 mmol) of 80% sodium hydride and 4.64 g (18.55 mmol) of benzyl tert.butyl malonate in 50 ml of dry dimethylformamide at 0°. The mixture was stirred for 6 hours at room temperature, left to stand at room temperature for 16 hours and evaporated to an oil. The residue in dichloromethane was washed twice with water and with saturated sodium chloride solution, dried over magnesium sulphate and evaporated. Chromatography on silica gel using ethyl acetate/hexane (1:4) for the elution yielded 6.08 g (64%) of dibenzyl 3(RS)-tert.butoxycarbonyl-2(RS)-isooctyl succinate as a pale amber oil.; Rf (ethyl acetate/hexane 1:3) 0.49.

(F) 6.02 g (11.8 mmol) of the triester from paragraph (E) in 90 ml of isopropanol was hydrogenated for 16 hours in the presence of 0.2 g of 10% palladium/carbon. The mixture was filtered, the filtrate was evaporated, toluene was added and the mixture was evaporated to remove traces of isopropanol. The residue in 75 ml of toluene and 1.65 ml (11.8 mmol) of triethylamine was refluxed for 2 hours and evaporated, and the residue was dissolved in ethyl acetate. The solution was washed with 0.5M hydrochloric acid and water, dried over magnesium sulphate and evaporated to yield 3.23 g (96%) of 4-tert.butyl hydrogen 2(RS)-isooctylsuccinate as an oil.

(G) 1.25 g (4.5 mmol) of N-tert.butoxycarbonyl-L-phenylalanine methylamide were dissolved in 15 ml of 4M hydrogen chloride in ethyl acetate, stirred at room temperature for 1 hour, evaporated and dried in vacuo. The residue in 10 ml of dry dimethylformamide was cooled to 0° and treated with 0.6 ml (4.7 mmol) of N-ethylmorpholine, a solution of 0.86 g (3 mmol) of 4-tert.butyl hydrogen 2(RS)-isooctylsuccinate in 15 ml of dichloromethane, 0.49 g (3.6 mmol) of hydroxybenzotriazole and 0.72 g (3.75 mmol) of 1-ethyl-3-(3-dimethylaminopropyl)-carbodiimide hydrochloride. The mixture was stirred for 1 hour at 0°, left to stand at 4° for 16 hours and evaporated. The residue in dichloromethane was washed in succession with 5% citric acid solution, water, 5% sodium hydrogen carbonate solution and saturated sodium chloride solution, dried over magnesium sulphate and evaporated. Chromatography on silica gel using ethyl acetate for the elution gave 1.17 g (87%) of [4-tert.butoxy 2(RS)-isooctylsuccinyl]-L-phenylalanine methylamide as an oil which solidified on standing; Rf (ethyl acetate/ hexane 1:1) 0.34 and 0.44.

(H) 1.02 g (2.28 mmol) of [4-tert.butoxy 2(RS)-isooctylsuccinyl)-L-phenylalanine methylamide were dissolved in 10 ml of trifluoroacetic acid and the solution was stirred for 1.5 hours and evaporated. The residue was evaporated three times with toluene, dissolved in chloroform, washed twice with water, dried and evaporated to give a solid. The solid was dissolved in 15 ml of dimethylformamide, cooled to 0° and treated with 0.6 ml (4.6 mmol) of N-ethylmorpholine, 0.37 g (2.7 mmol) of hydroxybenzotriazole, 0.54 g (2.8 mmol) of 1-ethyl-3-(3-dimethylaminopropyl)-carbodiimide hydrochloride and 0.42 g (3.4 mmol) of 0-benzylhydroxylamine. The mixture was stirred for 1 hour at 0° and for 16 hours at room temperature, evaporated and water was added. The solid which separated was washed in succession with 5% sodium hydrogen carbonate solution, water, 5% citric acid solution, water, 5% sodium hydrogen carbonate solution and water and then dried in vacuo. The solid obtained was dissolved in methanol/chloroform (1:1), and, after the addition of diethyl ether, there was obtained 0.71 g (63%) of [4-(N-benzyloxyamino)-2(RS)-isooctylsuccinyl]-L-phenylalanine methylamide as a white solid; MS:$(M+H)^+$ 496.

| Analysis for $C_{29}H_{41}N_3O_4$. 0.2 $CH_3OH$ | | | |
|---|---|---|---|
| Calc. | C, 69.85; | H, 8.39; | N, 8.37% |
| Found | C, 69.81; | H, 8.34; | N, 8.40%. |

Example 21

1.0 g (2.17 mmol) of [4-(N-benzyloxyamino)-2(R)-heptylsuccinyl]-L-neopentylglycine methylamide in 50 ml of methanol was hydrogenated for 1.5 hours in the presence of 0.33 g of 5% palladium/carbon. The mixture was filtered and the filtrate was evaporated to give 0.8 g (99%) of [4-(N-hydroxyamino)-2(R)-heptylsuccinyl]-L-neopentylglycine methylamide as a white solid; MS: $(M+H)^+$ 372.

| Analysis for $C_{19}H_{37}N_3O_4$. (371.52) | | | |
|---|---|---|---|
| Calc. | C, 61.43; | H, 10.04; | N, 11.31% |
| Found | C, 61.21; | H, 10.17; | N, 11.35%. |

The [4-(N-benzyloxyamino)-2(R)-heptylsuccinyl]-L-neopentylglycine methylamide used as the starting material was prepared as follows:

(A) 5.6 g (22.8 mmol) of tert.butoxycarbonyl-L-neopentylglycine were dissolved in 50 ml of dichloromethane at 0°. 1.85 ml (22.8 mmol) of pyridine and 9.67 g (22.8 mmol) of 70% di(1-benzotriazolyl)carbonate were added and the mixture was stirred at 0° for 1 hour. 3.94 ml (45.6 mmol) of a 40% aqueous solution of methylamine were added and the mixture was stirred for 1 hour at 0° and for 16 hours at room temperature. The solution was washed in succession with 5% sodium hydrogen carbonate solution, water, 2M hydrochloric acid solution, water, 5% sodium hydrogen carbonate solution and saturated sodium chloride solution, dried over magnesium sulphate and evaporated. Recrystallization from ethyl acetate yielded 5.26 g (89%) of tert.butoxycarbonyl-L-neopentylglycine methylamide as a white solid; Rf (ethyl acetate) 0.47.

(B) 4.84 g (18.8 mmol) of tert.butoxycarbonyl-L-neopentylglycine methylamide were stirred with 50 ml of 4M hydrogen chloride in ethyl acetate for 1 hour at room temperature and evaporated. Ethyl acetate was added, the solution was evaporated and the residue was dried in vacuo. The amine hydrochloride obtained was dissolved in 15 ml of dry dimethylformamide at 0°C, neutralized by the addition of 3.58 ml (28.2 mmol) of N-ethylmorpholine and added to a cooled (0°) solution of 4.25 g (15.6 mmol) of 4-tert.butyl hydrogen 2R-heptylsuccinate, prepared in a manner analogous to Example 1 (A-D) starting from D-$\alpha$-amino-nonanoic acid, 2.87 g (18.8 mmol) of hydroxybenzotriazole hydrate and 3.74 g (19.5 mmol) of 1-ethyl-3-(3-dimethylaminopropyl)-carbodiimide hydrochloride in 50 ml of dry dichloromethane at 0°. The mixture was stirred for 1 hour at 0° and for 16 hours at room temperature and then evaporated to an oil. The residue in ethyl acetate was washed with 5% citric acid solution, water, 5% sodium hydrogen carbonate solution and saturated sodium chloride solution, dried over magnesium sulphate and evaporated to an oil. Chromatography on silica gel using ethyl acetate/hexane (1:1) for the elution gave 5.13 g (80%) of [4-tert.butoxy 2(R)-heptylsuccinyl]-L-neopentylglycine methylamine as an oil; Rf (ethyl acetate:hexane 1:1) 0.49; MS: $(M+H)^+$ 413.

(C) 5.1 g (12.4 mmol) of [4-tert.butoxy 2(R)-heptylsuccinyl]-L-neopentylglycine methylamide were dissolved in 50 ml of trifluoroacetic acid and the solution was stirred for 2.5 hours and then evaporated. The residue was evaporated

twice with toluene and dissolved in dichloromethane. The solution was washed three times with water and with saturated sodium chloride solution, dried over magnesium sulphate and evaporated. The residue in 25 ml of dry dichloromethane was treated at 0° with 3.2 ml (25 mmol) of N-ethylmorpholine, 2.28 g (14.9 mmol) of hydroxybenzotriazole hydrate and 3.0 g (15.6 mmol) of 1-ethyl-3-(3-dimethylaminopropyl)-carbodiimide hydrochloride and the solution was stirred at 0° for 1 hour. 3.05 g (24.8 mmol) of O-benzylhydroxylamine were added and the mixture was stirred for 1 hour at 0° and for 16 hours at room temperature and then evaporated. Water was added and the solid obtained was washed in succession with 2M hydrochloric acid, water, 5% sodium hydrogen carbonate solution, water and hexane and dried in vacuo. The solid obtained was heated with diethyl ether and, after adding hexane and cooling, there were obtained 5.2 g (91%) of [4-(N-benzyloxyamino)-2(R)-heptylsuccinyl]-L-neopentylglycine methylamide as a hygroscopic gel which was dried in vacuo; MS: $(M+H)^+$ 462.

| Analysis for $C_{26}H_{43}N_3O_4$. 0.6 $H_2O$ | | | |
|---|---|---|---|
| Calc. | C, 66.10; | H, 9.43; | N, 8.89% |
| Found | C, 66.13; | H, 9.39; | N, 8.74%. |

Example 22

0.09 g (0.177 mmol) of [4-(N-benzyloxyamino)-2(R)-heptylsuccinyl]-(D or L)-β,β-dimethylphenylalanine methylamide (isomer 1) in 10 ml of methanol was hydrogenated for 1.5 hour in the presence of 30 mg of 5% palladium/carbon. The mixture was filtered, the filtrate was evaporated and the residue was dried in vacuo to give 0.075 g (100%) of [4-(N-hydroxyamino)-2(R)-heptylsuccinyl]-(D or L)-β,β-dimethylphenylalanine methylamide as a white foam; MS: $(M+H)^+$ 420.

| Analysis for $C_{23}H_{37}N_3O_4$. 0.4 $H_2O$ | | | |
|---|---|---|---|
| Calc. | C, 64.73; | H, 8.93; | N, 9.85% |
| Found | C, 64.81; | H, 8.77; | N, 10.14%. |

The [4-(N-benzyloxyamino)-2(R)-heptylsuccinyl)-(D or L)-β,β-dimethylphenylalanine methylamide (isomer 1) used as the starting material was prepared as follows:

(A) 2.3 g (11.9 mmol) of DL-β,β-dimethylphenylalanine were dissolved in 9 ml of water containing 0.48 g (12 mmol) of sodium hydroxide while stirring at 0°. 6.0 ml of 4M sodium hydroxide solution and 3.4 ml (23.8 mmol) of benzyl chloroformate were added dropwise over 0.3 hour. The mixture was stirred for 1 hour at 0° and for 16 hours at room temperature. The solution was extracted with diethyl ether, acidified to pH 2 with concentrated hydrochloric acid and the product was extracted into ethyl acetate. The organic solution was washed with saturated sodium chloride solution, dried over magnesium sulphate and evaporated to yield 3.86 g (99%) of N-benzyloxycarbonyl-DL-β,β-dimethylphenylalanine as an oil; Rf (System A) 0.54 MS: $(M+H)^+$ 328.

(B) 3.8 g (11.6 mmol) of N-benzyloxycarbonyl-DL-β,β-dimethylphenylalanine were converted into 2.45 g (62%) of N-benzyloxycarbonyl-DL-β,β-dimethylphenylalanine methylamide according to the procedure described in Example 21(A); Rf(ethyl acetate) 0.61; MS: $(M+H)^+$ 341.

(C) 2.4 g (7.06 mmol) of N-benzyloxycarbonyl-DL-β,β-dimethylphenylalanine methylamide were suspended in 30 ml of methanol and 3.5 ml of 2M hydrochloric acid and hydrogenated for 2 hours in the presence of 0.24 g of 5% palladium/carbon. The mixture was filtered, the filtrate was evaporated, the residue was evaporated three times with toluene and then dried in vacuo to give 1.75 g of DL-β,β-dimethylphenylalanine methylamide hydrochloride containing a trace of toluene; Rf (System A) 0.31; MS: $(M+H)^+$ 207.

(D) 0.485 g (2 mmol) of DL-β,β-dimethylphenylalanine methylamide hydrochloride was dissolved in 2 ml of dry dimethylformamide and the solution was cooled to 0°. 0.35 ml (2.8 mmol) of N-ethylmorpholine, 0.544 g (2 mmol) of 4-tert.butyl hydrogen 2(R)-heptylsuccinate and 0.46 g (2.4 mmol) of 1-ethyl-3-(3-dimethylaminopropyl)-carbodiimide hydrochloride were added and the mixture was stirred for 1 hour at 0° and for 64 hours at 4°. Water was added to the suspension and the white solid was filtered off, washed in succession with 2M hydrochloric acid, water, 5% sodium hydrogen carbonate solution and water and dried in vacuo to give 0.72 g (78%) of [4-tert.butoxy 2(R)-heptylsuccinyl]-DL-β,β-dimethylphenylalanine methylamide; Rf (ethyl acetate) 0.56 and 0.61; MS: $(M+H)^+$

461

(E) 0.71 g (1.54 mmol) of [4-tert.butoxy 2(R)-heptylsuccinyl]-DL-β,β-dimethylphenylalanine methylamide was dissolved in 7 ml of trifluoroacetic acid and the solution was stirred for 2.5 hours and evaporated. The residue was evaporated twice with toluene and dissolved in 30 ml of dichloromethane. The solution was washed with water, dried over magnesium sulphate and evaporated. The residue in 5 ml of dimethylformamide was treated at 0° with 0.31 g (2.02 mmol) of hydroxybenzotriazole hydrate and 0.37 g (1.93 mmol) of 1-ethyl-3-(3-dimethylaminopropyl)-carbodiimide hydrochloride and the solution was stirred at 0° for 1 hour. 0.38 g (3.08 mmol) of O-benzylhydroxylamine was added and the mixture was stirred for 1 hour at 0° and for 16 hours at room temperature and then evaporated. The residue in ethyl acetate was washed in succession with 5% sodium hydrogen carbonate solution, water, 2M hydrochloric acid, water, 5% sodium hydrogen carbonate solution and saturated sodium chloride solution, dried over magnesium sulphate and evaporated. Chromatography on silica gel using ethyl acetate/hexane (2:1), then ethyl acetate/hexane (3:1) and finally ethyl acetate for the elution gave [4-(N-benzyloxyamino) 2(R)-heptylsuccinyl] (D or L)-β,β-dimethylphenylalanine methylamide as two isomers. Isomer 1, (0.18 g; 23%): Rf (ethyl acetate/hexane 2:1) 0.32; MS: $(M+H)^+$ 510; and isomer 2 (0.27 g; 34%): Rf (ethyl acetate/hexane 2:1) 0.22; MS: $(M+H)^+$ 510.

Example 23

0.09 g (0.18 mmol) of [4-(N-benzyloxyamino)-2(R)-heptylsuccinyl](D or L)-threo-β-methylphenylalanine methylamide (isomer 1) in 10 ml of methanol was hydrogenated for 4 hours in the presence of 0.03 g of 5% palladium/carbon. The mixture was filtered, the filtrate was evaporated and the residue was dried in vacuo to give 0.072 g (98%) of [4-(N-hydroxyamino) 2(R)-heptylsuccinyl]-(D or L)-threo-β-methylphenylalanine methylamide as a white hygroscopic foam, MS: $(M+H)^+$ 406.

| Analysis for $C_{22}H_{35}N_3O_4$. 0.15 $H_2O$ | | | |
|---|---|---|---|
| Calc. | C, 64.73; | H, 8.72; | N, 10.29% |
| Found | C, 64.69; | H, 8.93; | N, 10.09%. |

The [4-(N-benzyloxyamino) 2(R)-heptylsuccinyl] (D or L)-threo-β-methylphenylalanine methylamide (isomer 1) used as the starting material was prepared from N-benzyloxycarbonyl-DL-threo-β-methylphenylalanine according to the procedure described in Example 22 (A)-(E). The isomers were separated by chromatography on silica gel using 1% methanol/dichloromethane, 2% methanol/dichloromethane and finally 3% methanol/dichloromethane for the elution. Isomer 1 had Rf (methanol/dichloromethane 1:19) 0.51 and MS: $(M+H)^+$ 496, and isomer 2 had Rf(methanol/dichloromethane 1:19) 0.41 and MS: $(M+H)^+$ 496.

Example 24

In a manner analogous to that described in the first paragraph of Example 22, from [4-(N-benzyloxyamino)-2(R)-heptylsuccinyl]-DL- erythro-β-methylphenylalanine methylamide there was prepared [4-(N-hydroxyamino)-2(R)-heptylsuccinyl]-DL-erythro-β-methylphenylalanine methylamide as a white hydroscopic foam: Rf (System B) 0.22 and 0.29; MS: $(M+H)^+$ 406.

| Analysis for $C_{22}H_{35}N_3O_4$. $0.3H_2O$. | | | |
|---|---|---|---|
| Calc.; | C, 64.30; | H, 8.73; | N, 10.23% |
| Found | C, 64.29; | H, 8.64; | N, 10.09% |

The starting material was prepared as follows:

In a manner analogous to that described in Example 22 (B-E), but starting with N-benzyloxycarbonyl-DL-erythro-β-methylphenylalanine there was obtained [4-(N-benzyloxyamino)-2(R)-heptylsuccinyl]-DL-erythro-β-methylphenylalanine methylamide as a white solid. Rf (methanol/dichloromethane 7:93) 0.40 and 0.45; MS: $(M+H)^+$ 496.

Example 25

0.12 g of [4-(N-benzyloxyamino)-2(R)-heptyl-3(R or S)-(phthalimidomethyl)succinyl]-L-tert.butyl glycine methylamide (diasteroisomer 1), prepared in a manner analogous to that described in Example 10(A)-(D), in 10 ml of methanol

was hydrogenated in the presence of 50 mg of 10% palladium/carbon for 16 hours. The catalyst was filtered off and the filtrate was evaporated to give a gelatinous solid. Purification on silica gel using 5% methanol in dichloromethane for the elution gave 0.06 g of [4-(N-hydroxyamino)-2(R)-heptyl-3(R or S)-(phthalimidomethyl)succinyl]-L-tert.butylglycine methylamide (diastereoisomer 1) as a white solid; Rf (10% methanol in dichloromethane) 0.34; MS: $(M+H)^+$ 517.

Example 26

0.17 g of [4-(N-benzyloxyamino)-2(R)-heptyl-3(R or S)-(3-phenylpropyl)succinyl]-L-leucyl-L-leucine ethylamide (diastereoisomer 1) in 10 ml of methanol was hydrogenated in the presence of 55 mg of palladium/carbon for 3 hours. The catalyst was filtered off and the filtrate was evaporated to give 0.13 g of [4-(N-hydroxyamino)-2(R)-heptyl-3(R or S)-(3-phenylpropyl)succinyl]-L-leucyl-L-leucine ethylamide (diasteroisomer 1) as a white solid; Rf (10% methanol in dichloromethane) 0.36; $MS(M+H)^+$ 603.

| Analysis for $C_{34}H_{58}N_4O_5$ (602.83) | | | |
|---|---|---|---|
| Calc. | C, 67.74; | H, 9.70; | N, 9.30% |
| Found | C, 68.00; | H, 9.93; | N, 9.20%. |

The starting material was prepared as follows:

In a manner analogous to that described in Example 10 (B)-(D), from 2.0 g of dibenzyl 3(RS)-tert.butyoxycarbonyl-2(R)-heptyl-succinate and 0.84 g of cinnamyl bromide there was obtained 0.17 g of [4-(N-benzyloxyamino)-2(R)-heptyl-3(R or S)-(3-phenylpropyl)succinyl]-L-leucyl]-L-leucine ethylamide (diastereoisomer 1) as an off-white solid; Rf (5% methanol in dichloromethane) 0.47; MS: $(M+H)^+$ 693.

Example 27

0.25 g of [4-(N-benzyloxyamino)-2(R)-heptyl-3(R or S)-[(3-methyl-2,5-dioxo-1-imidazolidinyl)methyl]succinyl]-L-leucyl-L-leucine ethylamide (diasteroisomer 1) in 25 ml of methanol was hydrogenated in the presence of 100 mg of 5% palladium/carbon for 6 hours. The catalyst was filtered off and the filtrate was evaporated to give 0.16 g of [4-(N-hydroxyamino)-2(R)-heptyl-3(R or S)-[(3-methyl-2,5-dioxo-1-imidazolidinyl)methyl]succinyl]-L-leucyl-L-lecuine ethylamide (diastereomer 1) as a white solid; Rf (10% methanol in dichloromethane) 0.25; MS: $(M+H)^+$ 611.

| Analysis for $C_{30}H_{54}N_6O_7$ 0.6 $H_2O$ | | | |
|---|---|---|---|
| Calc. | C, 57.91; | H, 8.95; | N, 13.51% |
| Found | C, 58.17; | H, 8.99; | N, 13.23%. |

The starting material was prepared as follows:

In a manner analogous to that described in Example 10 (B)-(D), from 2.0 g of dibenzyl-3(RS)-tert.butoxycarbonyl-2(R)-heptylsuccinate and 0.88 g of 3-bromomethyl-1-methylhydantoin there was obtained 0.28 g of [4-(N-benzyloxyamino)-2(R)-heptyl-3(R or S)-[(3-methyl-2,5 dioxo-1-imidazolidinyl)methyl]succinyl]-L-leucyl-L-leucine ethylamide (diastereoisomer 1); Rf(10% methanol in dichloromethane) 0.7; MS: $(M+H)^+$ 701.

The following Examples illustrate pharmaceutical preparations containing the hydroxamic acid derivatives provided by the present invention:

Example A

Tablets containing the following ingredients may be produced in a conventional manner:

| Ingredient | Per tablet |
|---|---|
| Hydroxamic acid derivative | 10.0 mg |
| Lactose | 125.0 mg |
| Corn starch | 75.0 mg |
| Talc | 4.0 mg |
| Magnesium stearate | 1.0 mg |
| Total weight | 215.0 mg |

Example B

Capsules containing the following ingredients may be produced in a conventional manner:

| Ingredient | Per capsule |
|---|---|
| Hydroxamic acid derivative | 10.0 mg |
| Lactose | 165.0 mg |
| Corn starch | 20.0 mg |
| Talc | 5.0 mg |
| Capsule fill weight | 200.0 mg |

**Claims**

1. Compounds of the general formula

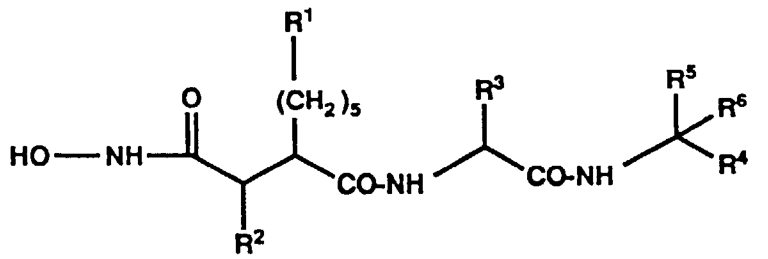

(I)

wherein

| | |
|---|---|
| $R^1$ | represents 2-7C straight-chain or branched-chain alkyl; |
| $R^2$ | represents hydrogen, 1-6C alkyl or a group of the formula $-(CH_2)_n$-aryl or $-(CH_2)_n$-Het in which n stands for 1-4 and Het represents a 5- or 6-membered N-heterocyclic ring which (a) is attached via the N atom, (b) optionally contains N, O and/or S as additional hetero atom(s) in a position or positions other than adjacent to the linking N atom, (c) is substituted by oxo on one or both C atoms adjacent to the linking N atom and (d) is optionally benz-fused or optionally substituted on one or more other carbon atoms by 1-6C alkyl or oxo and/or on any additional N atom(s) by 1-6C alkyl; |
| $R^3$ | represents the characterizing group of a natural or non-natural $\alpha$-amino acid in which any functional group present may be protected, with the proviso that $R^3$ does not represent hydrogen or bicyclo aryl methylene; |
| $R^4$ | represents carboxyl, (1-6C alkoxy)carbonyl, carbamoyl or (1-6C alkyl)carbamoyl; |
| $R^5$ | represents the characterizing group of a naturally occurring $\alpha$-amino acid in which any functional group present may be protected; and |
| $R^6$ | represents hydrogen; or |
| $R^4$, $R^5$ and $R^6$ | each individually represent hydrogen or 1-6C alkyl, |

and pharmaceutically acceptable salts thereof.

2. Compounds according to claim 1, wherein $R^2$ represents hydrogen, 1-6C alkyl or a group of the formula $-(CH_2)_n$-Het and $R^6$ represents hydrogen.

3. Compounds according to claim 1 or claim 2, wherein $R^1$ represents ethyl, isopropyl, n-butyl or n-hexyl.

4. Compounds according to claim 3, wherein $R^1$ represents ethyl.

5. Compounds according to any one of claims 1 to 4, wherein $R^2$ represents hydrogen, methyl, a group of the formula $-(CH_2)_n$-aryl in which the aryl group is phenyl or a group of the formula $-(CH_2)_n$-Het in which Het represents a 5-

or 6-membered N-heterocyclic ring which optionally contains as additional hetero atom(s) one or two N atoms, one N atom and one O atom or one O atom.

6. Compounds according to claim 5, wherein Het represents a group of the formula

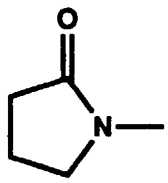

(a)

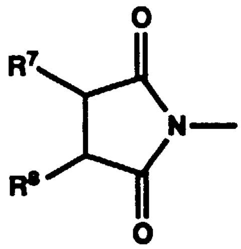

(b)

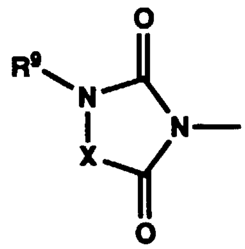

(c)

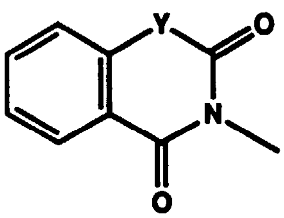

(d)

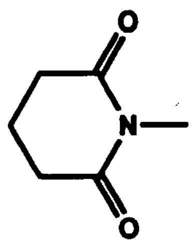

(e)

or

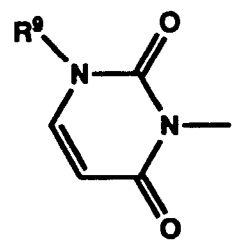

(f)

in which

| | |
|---|---|
| $R^7$ and $R^8$ | each represent hydrogen or together represent an additional bond or the remainder of a fused benzene ring; |
| $R^9$ | represents hydrogen or 1-6C alkyl; |
| X | represents -CO-, $-CH_2-$, -CH(1-6C alkyl)-, $-C(1\text{-}6C\ alkyl)_2$, -NH-, -N(1-6C alkyl)- or -O-; and |
| Y | represents -O-, -NH- or -N(1-6C alkyl)-. |

7. Compounds according to claim 6, wherein Het represents a group of formula (b) or (c).

8. Compounds according to claim 7, wherein Het represents phthalimido or 3-methyl-2,5-dioxo-1-imidazolidinyl.

9. Compounds according to any one of claims 1 to 8, wherein n stands for 1 when $R^1$ represents a group of the formula $-(CH_2)_n$-Het and for 3 when $R^1$ represents a group of the formula $-CH_2)_n$-aryl.

10. Compounds according to any one of claims 1 to 9, wherein $R^3$ represents methyl, isobutyl, tert.butyl, protected 4-aminobutyl, neopentyl, n-heptyl, cyclohexylmethyl, benzyl, α-methylbenzyl or α,α-dimethylbenzyl.

11. Compounds according to claim 10, wherein the protected 4-aminobutyl group is 4-phthalimidobutyl.

12. Compounds according to any one of claims 1 to 11, wherein $R^4$ represents (1-6C alkyl)carbamoyl, $R^5$ represents isobutyl and $R^6$ represents hydrogen.

13. Compounds according to claim 12, wherein $R^4$ represents methylcarbamoyl or ethylcarbamoyl.

14. Compounds according to any one of claims 1 to 11, wherein $R^4$, $R^5$ and $R^6$ each represent hydrogen or $R^4$ and $R^5$ each represent hydrogen and $R^6$ represents tert.butyl or $R^4$, $R^5$ and $R^6$ each represent methyl.

**15.** [4-(N-Hydroxyamino)-2(R or S)-heptylsuccinyl]-L-leucyl-L-leucine ethylamide.

**16.** [4-(N-Hydroxyamino)-2(R or S)-nonylsuccinyl]-L-leucyl-L-leucine ethylamide.

**17.** [4-(N-Hydroxyamino)-2(R or S)-heptyl-3(S)-methylsuccinyl]L-leucyl-L-leucine ethylamide.

**18.** [4-(N-Hydroxyamino)-2(R)-heptyl-3(R or S)-(phthalimidomethyl)succinyl]-L-leucyl-L-leucine ethylamide.

**19.** [4-(N-Hydroxyamino)-2(RS)-nonylsuccinyl]-L-tert.butylglycine methylamide.

**20.** [4-(N-Hydroxyamino)-2(RS)-heptylsuccinyl]-L-phenylalanine methylamide.

**21.** [4-(N-Hydroxyamino)-2(R)-heptyl-3(R or S)-(phthalimidomethyl)succinyl]-L-tert.butylglycine methylamide.

**22.** [4-(N-Hydroxyamino)-2(R)-heptyl-3(R or S)-(3-phenylpropyl)succinyl]-L-leucyl-L-leucine ethylamide.

**23.** A compound according to claim 2 selected from:

[4-(N-Hydroxyamino)-2(RS)-heptylsuccinyl]-L-leucine methylamide,
[4-(N-hydroxyamino)-2(RS)-heptylsuccinyl]-L-leucine neopentylamide,
[4-(N-hydroxyamino)-2(RS)-heptylsuccinyl]-L-alanyl-L-leucine ethylamide,
[4-(N-Hydroxyamino)-2(RS)-heptylsuccinyl]-L-($N^{\varepsilon}$-phthaloyl)-lysylL-leucine ethylamide,
[4-(N-hydroxyamino)-2(RS)-undecylsuccinyl]-L-leucyl-L-leucine ethylamide and
[4-(N-hydroxyamino)-2(RS)-heptylsuccinyl]-L-phenylalanyl-L-leucine ethylamide.

**24.** A compound according to claim 1 selected from:

[4-(N-Hydroxyamino)-2(RS)-heptylsuccinyl]-L-nonalyl-L-leucine ethylamide,
[4-(N-hydroxyamino)-2(RS)-heptylsuccinyl]-L-phenylalanine tert.butylamide,
[4-(N-hydroxyamino)-2(RS)-heptylsuccinyl]-L-tertbutylglycine methylamide,
[4-(N-hydroxyamino)-2(RS)-heptylsuccinyl]-L-neopentylglycine methylamide,
[4-(N-hydroxyamino)-2(RS)-heptylsuccinyl]-L-homophenylalanyl-L-leucine methylamide,
[4-(N-hydroxyamino)-2(RS)-heptylsuccinyl]-L-cyclohexylalanine methylamide,
[4-(N-hydroxyamino)-2(RS)-isooctylsuccinyl]-L-phenylalanine methylamide,
[4-(N-hydroxyamino)-2(R)-heptylsuccinyl]-L-neopentylglycine methylamide,
[4-(N-hydroxyamino)-2(R)-heptylsuccinyl]-(D or L)-β,β-dimethylphenylalanine methylamide,
[4-(N-hydroxyamino) 2(R)-heptylsuccinyl]-(D or L)-threo-β-methylphenylalanine methylamide,
[4-(N-hydroxyamino) 2(R)-heptylsuccinyl]-DL-erythro-β-methylphenylalanine methylamide and
[4-(N-hydroxyamino)-2(R)-heptyl-3(R or S)-[(3-methyl-2,5-dioxo-1-imidazolidinyl)methyl]succinyl]-L-leucyl-L-leucine ethylamide.

**25.** Compounds of the general formula

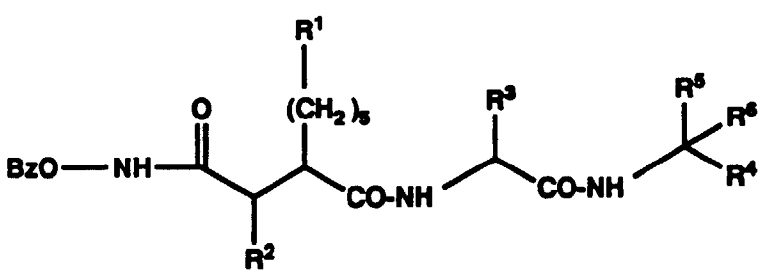

(II)

wherein $R^1$, $R^2$, $R^3$, $R^4$, $R^5$ and $R^6$ have the significance given in claim 1 and Bz represents benzyl.

**26.** Compounds of the general formula

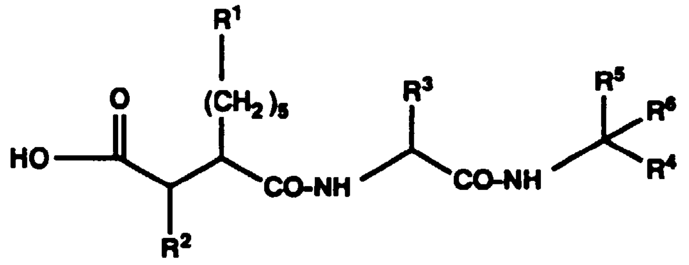

(III)

wherein $R^1$, $R^2$, $R^3$, $R^4$, $R^5$ and $R^6$ have the significance given in claim 1, and activated esters thereof.

**27.** Compounds according to any one of claims 1-24 for use as therapeutically active substances.

**28.** Compounds according to any one of claims 1-24 for use in the control or prevention of degenerative joint diseases or in the treatment of invasive tumours, atherosclerosis or multiple sclerosis.

**29.** A process for the manufacture of the compounds claimed in any one of claims 1-24, which process comprises

(a) catalytically hydrogenating a compound of the general formula

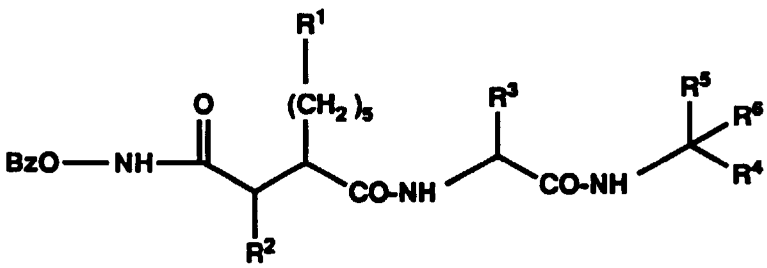

(II)

wherein $R^1$, $R^2$, $R^3$, $R^4$, $R^5$ and $R^6$ have the significance given in claim 1 and Bz represents benzyl, or

(b) reacting an acid of the general formula

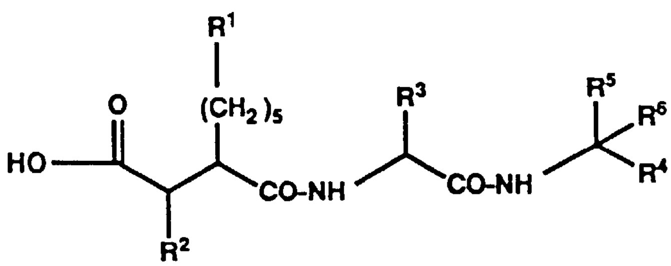

(III)

wherein $R^1$, $R^2$, $R^3$, $R^4$, $R^5$ and $R^6$ have the significance given in claim 1,

or an activated ester thereof with a compound of the general formula

$$H_2N\text{-}OZ \qquad (IV)$$

wherein Z represents hydrogen, tri(1-6C alkyl)silyl or diphenyl(1-6C alkyl)silyl,
and, where required, cleaving off any diphenyl(1-6C alkyl)silyl group present in the reaction product, and, if desired, converting a compound of formula I obtained into a pharmaceutically acceptable salt.

**30.** A medicament containing a compound according to any one of claims 1-24 and a therapeutically inert carrier material.

**31.** A medicament for the control or prevention of degenerative joint diseases or for the treatment of invasive tumours, atherosclerosis or multiple sclerosis, containing a compound according to any one of claims 1-24 and a therapeutically inert carrier material.

**32.** The use of a compound according to any one of claims 1-24 for the manufacture of a medicament for the control or prevention of degenerative joint diseases or for the treatment of invasive tumours, atherosclerosis or multiple sclerosis.

**Patentansprüche**

**1.** Verbindungen der allgemeinen Formel

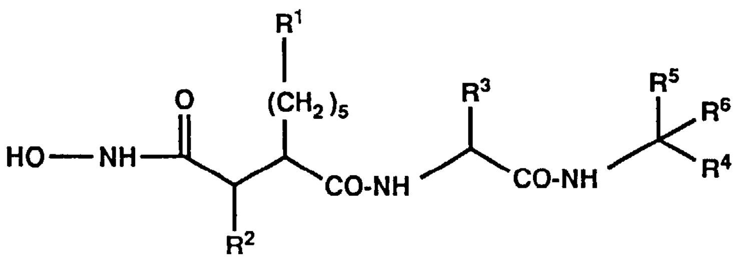

(I)

worin

| | |
|---|---|
| $R^1$ | 2-7C gradkettiges oder verzweigtes Alkyl bedeutet; |
| $R^2$ | Wasserstoff, 1-6C Alkyl oder eine Gruppe der Formel $-(CH_2)_n$-Aryl oder $-(CH_2)_n$-Het bedeutet in welcher n für 1-4 steht und Het einen 5- oder 6 gliedrigen N-heterozyklischen Ring bedeutet, welcher (a) über das N-Atom verknüpft ist, (b) gegebenenfalls N, O und/oder S als zusätzliche (s) Heteroatom(e) in einer dem verknüpfenden N-Atom nicht benachbarten Position oder Positionen enthält, (c) an einer oder beiden dem verknüpfenden N-Atom benachbarten C-Atomen durch Oxo substituiert ist und (d) gegebenenfalls mit Benzol fusioniert ist oder gegebenenfalls an einem oder mehreren anderen Kohlenstoffatomen durch 1-6C Alkyl oder Oxo und/oder an jedem zusätzlichen N-Atom durch 1-6C Alkyl substituiert ist; |
| $R^3$ | die charakterisierende Gruppe einer natürlichen oder nichtnatürlichen $\alpha$-Aminosäure bedeutet, in welcher jegliche vorhandene funktionelle Gruppe geschützt sein kann, mit der Bedingung, dass $R^3$ nicht Wasserstoff oder Bicycloarylmethylen bedeutet; |
| $R^4$ | Carboxyl, (1-6C Alkoxy)carbonyl, Carbamoyl oder (1-6C Alkyl)carbamoyl bedeutet; |
| $R^5$ | die charakterisierende Gruppe einer natürlichen vorkommenden $\alpha$-Aminosäure bedeutet, in welcher jegliche vorhandene funktionelle Gruppe geschützt sein kann; |
| $R^6$ | Wasserstoff bedeutet; oder |
| $R^4$, $R^5$ und $R^6$ | jeweils für sich Wasserstoff oder 1-6C Alkyl bedeuten, |

und pharmakologisch verträgliche Salze davon.

**2.** Verbindungen gemäss Anspruch 1, worin $R^2$ Wasserstoff, 1-6C Alkyl oder eine Gruppe der Formel $-(CH_2)_n$-Het bedeutet und $R^6$ Wasserstoff bedeutet.

**3.** Verbindungen gemäss Anspruch 1 der Anspruch 2, worin $R^1$ Ethyl, Isopropyl, n-Butyl oder n-Hexyl bedeutet.

**4.** Verbindungen gemäss Anspruch 3, worin $R^1$ Ethyl bedeutet.

**5.** Verbindungen gemäss einem der Ansprüche 1 bis 4, worin $R^2$ Wasserstoff, Methyl eine Gruppe der Formel $-(CH_2)_n-$Aryl bedeutet in welcher die Arylgruppe Phenyl oder eine Gruppe der Formel $-(CH_2)_n-$Het ist, in welcher Het einen 5- oder 6-gliederigen N-heteroczyklischen Ring bedeutet, welcher gegebenenfalls als zusätzliche Heteroatome ein oder zwei N-Atome, ein N Atom und ein O Atom oder ein O Atom enthält.

**6.** Verbindungen gemäss Anspruch 5, worin Het eine Gruppe der Formel

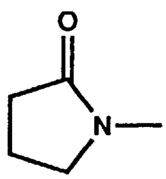

(a)

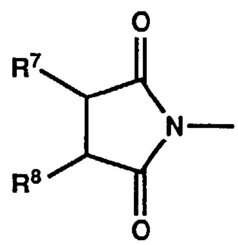

(b)

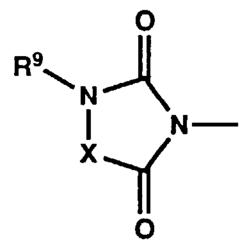

(c)

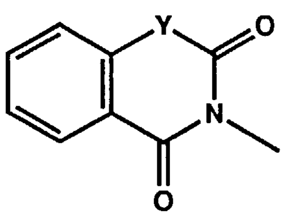

(d)

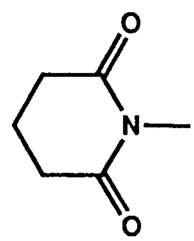

(e)

or

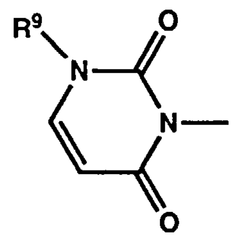

(f)

bedeutet in welcher

| | |
|---|---|
| $R^7$ und $R^8$ | jeweils Wasserstoff bedeuten oder zusammen eine zusätzliche Bindung oder den Rest eines ankondensierten Benzolrings bedeuten; |
| $R^9$ | Wasserstoff oder 1-6C Alkyl bedeutet; |
| X | represents -CO-, $-CH_2-$, -CH(1-6C Alkyl)-, $-C(1\text{-}6C\ Alkyl)_2-$, -NH-, -N(1-6C Alkyl)- oder -O- bedeutet; und |
| Y | -O-, -NH- oder -N(1-6C Alkyl)- bedeutet. |

**7.** Verbindungen gemäss Anspruch 6, worin Het eine Gruppe der Formel (b) oder (c) bedeutet.

**8.** Verbindungen gemäss Anspruch 7, worin Het Phthalimido oder 3-Methyl-2,5-dioxo-1-imidazolidinyl bedeutet.

**9.** Verbindungen gemäss einem der Ansprüche 1 bis 9, worin n 1 ist wenn $R^1$ eine Gruppe der Formel $-(CH_2)_n-$Het bedeutet und n 3 ist wenn $R^1$ eine Gruppe der Formel $-(CH_2)_n-$Aryl bedeutet.

**10.** Verbindungen gemäss einem der Ansprüch 1 bis 9, worin $R^3$ Methyl, Isobutyl, tert.Butyl, geschütztes 4-Aminobutyl, Neopentyl, n-Heptyl, Cyclohexylmethyl, Benzyl, α-Methylbenzyl oder α,α-Dimethylbenzyl bedeutet.

**11.** Verbindungen gemäss Anspruch 10, worin die geschützte 4-Aminobutyl Gruppe 4-Phthalimidobutyl ist.

**12.** Verbindungen gemäss einem der Ansprüche 1 bis 11, worin $R^4$ (1-6C Alkyl)carbamoyl, $R^5$ Isobutyl und $R^6$ Wasserstoff bedeutet.

**13.** Verbindungen gemäss Anspruch 12, worin $R^4$ Methylcarbamoyl oder Ethylcarbamoyl bedeutet.

**14.** Verbindungen gemäss einem der Ansprüche 1 bis 11, worin $R^4$, $R^5$ und $R^6$ jeweils Wasserstoff bedeuten oder $R^4$ und $R^5$ jeweils Wasserstoff und $R^6$ tert.Butyl bedeuten oder $R^4$, $R^5$ und $R^6$ jeweils Methyl bedeuten.

**15.** [4-(N-Hydroxyamino)-2(R or S)-heptylsuccinyl]-L-leucyl-L-leucin ethylamid.

**16.** [4-(N-Hydroxyamino)-2(R or S)-nonylsuccinyl]-L-leucyl-L-leucin ethylamid.

**17.** [4-(N-Hydroxyamino)-2(R or S)-heptyl-3(S)-methylsuccinyl]-L-leucyl-L-leucin ethylamid.

**18.** [4-(N-Hydroxyamino)-2(R)-heptyl-3(R or S)-(phthalimidomethyl)succinyl]-L-leucyl-L-leucin ethylamid.

**19.** [4-(N-Hydroxyamino)-2(RS)-nonylsuccinyl]-L-tert.butylglycin methylamid.

**20.** [4-(N-Hydroxyamino)-2(RS)-heptylsuccinyl]-L-phenylalanin methylamid.

**21.** [4-(N-Hydroxyamino)-2(R)-heptyl-3(R or S)-(phthalimidomethyl)succinyl]-L-tert.butylglycin methylamid.

**22.** [4-(N-Hydroxyamino)-2(R)-heptyl-3(R or S)-(3-phenylpropyl)succinyl]-L-leucyl-L-leucin ethylamid.

**23.** Eine Verbindung gemäss Anspruch 2 ausgewählt aus:

[4-(N-Hydroxyamino)-2(RS)-heptylsuccinyl]-L-leucin methylamid,
[4-(N-Hydroxyamino)-2(RS)-heptylsuccinyl]-L-leucin neopentylamid,
[4-(N-Hydroxyamino)-2(RS)-heptylsuccinyl]-L-alanyl-L-leucin ethylamid,
[4-(N-Hydroxyamino )-2(RS)-heptylsuccinyl]-L-($N^{\varepsilon}$-phthaloyl)-lysyl-L-leucin ethylamid,
[4-(N-Hydroxyamino)-2(RS)-undecylsuccinyl]-L-leucyl-L-leucin ethylamid und
[4-(N-Hydroxyamino)-2(RS)-heptylsuccinyl]-L-phenylalanyl-L-leucin ethylamid.

**24.** Eine Verbindung gemäss Anspruch 1 ausgewählt aus:

[4-(N-Hydroxyamino)-2(RS)-heptylsuccinyl]-L-nonalyl-L-leucin ethylamid,
[4-(N-Hydroxyamino)-2(RS)-heptylsuccinyl]-L-phenylalanin tert.butylamid,
[4-(N-Hydroxyamino)-2(RS)-heptylsuccinyl]-L-tertbutylglycin methylamid,
[4-(N-Hydroxyamino)-2(RS)-heptylsuccinyl]-L-neopentylglycin methylamid,
[4-(N-Hydroxyamino)-2(RS)-heptylsuccinyl]-L-homophenylalanyl-L-leucin ethylamid,
[4-(N-Hydroxyamino)-2(RS)-heptylsuccinyl]-L-cyclohexylalanin methylamid,
[4-(N-Hydroxyamino)-2(RS)-isooctylsuccinyl]-L-phenylalanin methylamid,
[4-(N-Hydroxyamino)-2(R)-heptylsuccinyl]-L-neopentylglycin methylamid,
[4-(N-Hydroxyamino)-2(R)-heptylsuccinyl]-(D or L)-β,β-dimethylphenylalanin methylamid,
[4-(N-Hydroxyamino) 2(R)-heptylsuccinyl]-(D or L)-threo-β-methylphenylalanin methylamid,
[4-(N-Hydroxyamino) 2(R)-heptylsuccinyl]-DL-erthro-β-methylphenylalanin methylamid und
[4-(N-Hydroxyamino)-2(R)-heptyl-3(R or S)-[(3-methyl-2,5-dioxo-1-imidazolidinyl)methyl]succinyl]-L-leucyl-L-leucin ethylamid.

**25.** Verbindungen der allgemeinen Formel

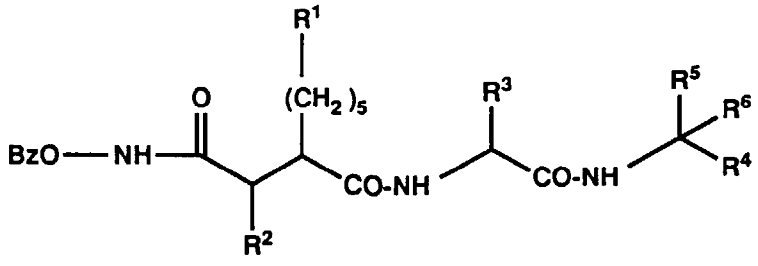

(II)

worin $R^1$, $R^2$, $R^3$, $R^4$, $R^5$ und $R^6$ die in Anspruch 1 angegebene Bedeutung haben und Bz Benzyl bedeutet.

**26.** Verbindungen der allgemeinen Formel

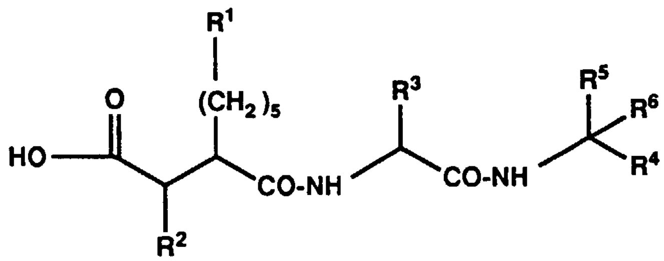

(III)

worin $R^1$, $R^2$, $R^3$, $R^4$, $R^5$ und $R^6$ die in Anspruch 1 angegebene Bedeutung haben und Aktivester davon.

**27.** Verbindungen gemäss einem der Ansprüche 1-24 zur Verwendung als therapeutisch aktive Substanzen.

**28.** Verbindungen gemäss einem der Ansprüche 1-24 zur Verwendung in der Kontrolle oder Vorbeugung von degenerativen Gelenkerkrankungen oder in der Behandlung von invasiven Tumoren, Atherosclerose oder Multipler Sclerose.

**29.** Ein Verfahren zur Herstellung der in den Ansprüchen 1 bis 24 beanspruchten Verbindungen, umfassed

(a) katalytische Hydrogenierung einer Verbindung der allgemeinen Formel

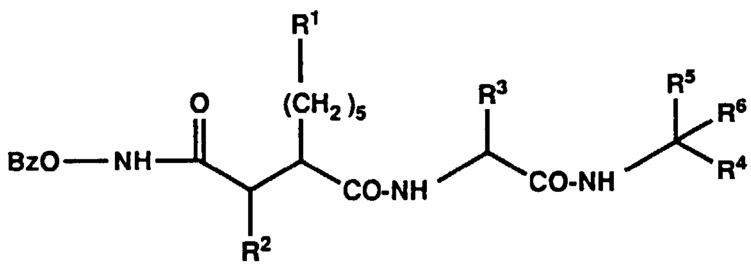

(II)

worin $R^1$, $R^2$, $R^3$, $R^4$, $R^5$ und $R^6$ die in Anspruch 1 angegebene Bedeutung haben und Bz Benzyl bedeutet, oder

(b) reagieren einer Säure der allgemeinen Formel

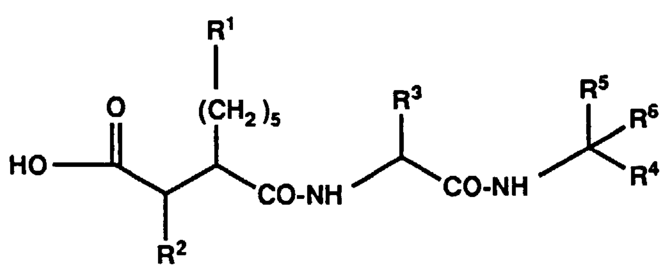

(III)

worin $R^1$, $R^2$, $R^3$, $R^4$, $R^5$ und $R^6$ die in Anspruch 1 gegebene Bedeutung haben,

oder ein aktivierter Ester davon, mit einer Verbindung der allg. Formel

$$H_2N\text{-}OZ \qquad (IV)$$

worin Z Wasserstoff, tri(1-6C Alkyl)silyl or Diphenyl(1-6C Alkyl)silyl bedeutet,

und, falls erforderlich, abspalten von jeglicher Diphenyl(1-6C Alkyl)silyl Gruppe die im Reaktionsprodukt vorhanden ist, und,

falls gewünscht, umwandeln einer mit der Formel I erhaltenen Verbindung in ein pharmazeutisch akzeptables Salz.

**30.** Ein Medikament enthaltend eine Verbindung gemäss einem der Ansprüche 1-24 und ein therapeutisch innertes Trägermaterial.

**31.** Ein Medikament für die Kontrolle oder Vermeidung von degenerativen Gelenkerkrankungen oder für die Behandlung von invasiven Tumoren, Atherosclerose oder Multipler Sclerose, enthaltend eine Verbindung gemäss einem der Ansprüche 1-24 und ein therapeutisch inertes Trägermaterial.

**32.** Verwendung einer Verbindung gemäss einem der Ansprüche 1-24 zur Herstellung eines Medikamentes für die Kontrolle oder Vermeidung von degenerativen Gelenkerkrankungen oder für die Behandlung von invasiven Tumoren, Atherosclerose oder Multipler Sclerose.

**Revendications**

**1.** Composés de la formule générale

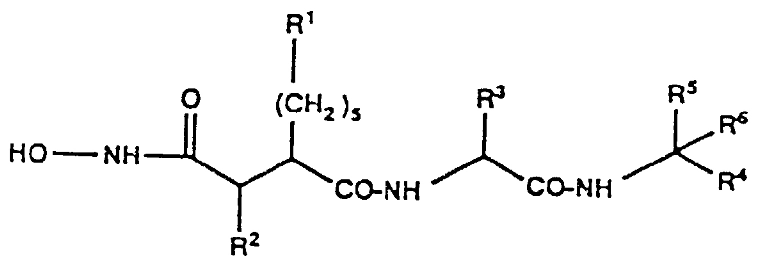

(I)

dans laquelle :

$R^1$ représente un alkyle en $C_2$ à $C_7$, à chaîne droite ou à chaîne ramifiée;

$R^2$ représente de l'hydrogène, un alkyle en $C_1$ à $C_6$ ou bien un groupe de la formule $-(CH_2)_n$-aryle ou $-(CH_2)_n$-Het dans laquelle n représente de 1 à 4 et Het représente un noyau N-hétérocyclique à 5 ou 6 éléments, qui (a) est rattaché par l'intermédiaire de l'atome N, (b) renferme facultativement N,O et/ou S en tant qu'hétéroatome(s) supplémentaire(s) en une position ou en des positions autres que celles adjacentes à l'atome N de liaison, (c) est substitué par un oxo sur un ou deux atomes C adjacents à l'atome N de liaison et (d) est facultativement fusionné à un noyau benzénique ou facultativement substitué sur un ou plus d'un autre atome de carbone par un alkyle en $C_1$ à $C_6$ ou un oxo et/ou sur un (des) atome(s) N supplémentaire(s) par un alkyle en $C_1$ à $C_6$;

$R^3$ représente le groupe caractérisant d'un acide $\alpha$-amino, naturel ou non naturel, dans lequel un groupe fonctionnel quelconque présent peut être protégé, sous la condition que $R^3$ ne représente pas de l'hydrogène ou un bicycloaryl-méthylène;

$R^4$ représente un radical carboxyle, (alcoxy en $C_1$ à $C_6$) carbonyle, carbamoyle ou (alkyl en $C_1$ à $C_6$) carbamoyle;

$R^5$ représente le groupe caractérisant d'un acide $\alpha$-aminé, se trouvant à l'état naturel, dans lequel un groupe fonctionnel quelconque présent peut être protégé ;

$R^6$ représente de l'hydrogène; ou

$R^4$, $R^5$ et $R^6$ représentent chacun, individuellement, de l'hydrogène ou un alkyle en $C_1$ à $C_6$, ainsi que leurs sels pharmaceutiquement acceptables.

**2.** Composés selon la revendication 1, dans lesquels $R^2$ représente de l'hydrogène, un alkyle en $C_1$ à $C_6$ où un

groupe de la formule $-(CH_2)_n$-Het et $R^6$ représente de l'hydrogène.

**3.** Composés selon la revendication 1 ou la revendication 2, dans lesquels $R^1$ représente un radical éthyle, isopropyle, n-butyle ou n-hexyle.

**4.** Composés selon la revendication 3, dans lesquels $R^1$ représente de l'éthyle.

**5.** Composés selon l'une quelconque des revendications 1 à 4, dans lesquels $R^2$ représente de l'hydrogène, du méthyle, un groupe de la formule $-(CH_2)_n$-aryle dans lesquels le groupe aryle est du phényle ou un groupe de la formule $-(CH_2)_n$-Het dans laquelle Het représente un noyau N-hétérocyclique à 5 ou 6 éléments, qui facultativement renferme comme hétéroatome (s) supplémentaire(s), un ou deux atomes N, un atome N et un atome O ou bien un atome O.

**6.** Composés selon la revendication 5, dans lesquels Het représente un groupe choisi dans les formules

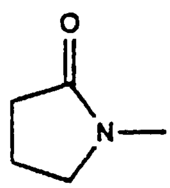

(a)

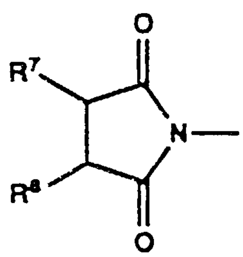

(b)

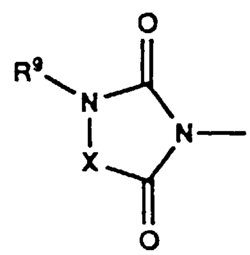

(c)

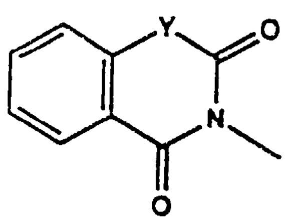

(d)

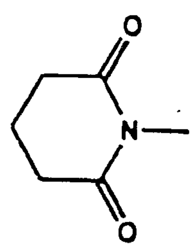

(e)

ou

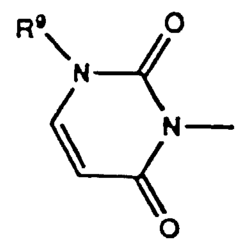

(f)

dans laquelle

$R^7$ et $R^8$ représentent chacun de l'hydrogène ou représentent conjointement une liaison supplémentaire ou bien le reste d'un noyau benzénique fusionné;
$R^9$ représente de l'hydrogène ou un alkyle en $C_1$ à $C_6$;
X représente -CO-, $-CH_2-$, -CH(alkyl en $C_1$ à $C_6$), -C(alkyl en $C_1$ à $C_6)_2$-, -NH-, -N(alkyl en $C_1$ à $C_6$)- ou -O-; et
Y représente -O-, -NH- ou -N(alkyl en $C_1$ à $C_6$)-.

**7.** Composés selon la revendication 6, dans lesquels Het représente un groupe de la formule (b) ou (c).

**8.** Composés selon la revendication 7, dans lesquels Het représente un radical phtalimido ou 3-méthyl-2,5-dioxo-1-imidazolidinyle.

**9.** Composés selon l'une quelconque des revendications 1 à 8, dans lesquels n représente 1 lorsque $R^1$ représente un groupe de la formule $-(CH_2)_n$-Het et est 3 lorsque $R^1$ représente un groupe de la formule $-(CH_2)_n$-aryle.

**10.** Composés selon l'une quelconque des revendications 1 à 9, dans lesquels $R^3$ représente un radical méthyle,

isobutyle, tert-butyle, 4-aminobutyle protégé, néopentyle, n-heptyle, cyclohexylméthyle, benzyle, α-méthylbenzyle ou α,α-diméthylbenzyle.

**11.** Composés selon la revendication 10, dans lesquels le groupe 4-aminobutyle protégé est le 4-phtalimidobutyle.

**12.** Composés selon l'une quelconque des revendications 1 à 11, dans lesquels $R^4$ représente un (alkyl en $C_1$ à $C_6$) carbamoyle, $R^5$ représente de l'isobutyle et $R^6$ représente de l'hydrogène.

**13.** Composés selon la revendication 12, dans lesquels $R^4$ représente du méthylcarbamoyle ou de l'éthylcarbamoyle.

**14.** Composés selon l'une quelconque des revendications 1 à 11, dans lesquels $R^4$, $R^5$ et $R^6$ représentent chacun de l'hydrogène ou bien $R^4$ et $R^5$ représentent chacun de l'hydrogène et $R^6$ représente du tert-butyle ou bien $R^4$, $R^5$ et $R^6$ représentent chacun du méthyle.

**15.** Le [4-(N-hydroxyamino)-2(R ou S)-heptylsuccinyl] -L-leucyl-L-leucine éthylamide.

**16.** Le [4-(N-hydroxyamino)-2(R ou S)-nonylsuccinyl]L-leucyl-L-leucine éthylamide.

**17.** Le [4-(N-hydroxyamino)-2(R ou S)-heptyl-3(S)méthylsuccinyl]-L-leucyl-L-leucine éthylamide.

**18.** Le [4-(N-hydroxyamino)-2(R)-heptyl-3(R ou S)-(phtalimidométhyl)succinyl]-L-leucyl-L-leucine éthylamide.

**19.** Le [4-(N-hydroxyamino)-2(RS)-nonylsuccinyl]-L-tert-butyl-glycine méthylamide.

**20.** Le [4-(N-hydroxyamino)-2(RS)-heptylsuccinyl]-L-phényl-alanine méthylamide.

**21.** Le [4-(hydroxyamino)-2-(R)-heptyl-3(R ou S)-(phtalimido-méthyl)succinyl]-L-tert-butylglycine méthylamide.

**22.** Le [4- (N-hydroxyamino) -2 (R) -heptyl-3(R ou S)-(3-phénylpropyl)-succinyl]-L-leucyl-L-leucine éthylamide.

**23.** Un composé selon la revendication 2 choisi parmi:

le [4-(N-hydroxyamino) -2 (RS) -heptylsuccinyl]-L-leucine méthylamide.
le [4-(N-hydroxyamino)-2(RS)-heptylsuccinyl]-L-leucine néopentylamide.
le [4-(N-hydroxyamino)-2(RS)-heptylsuccinyl]-L-alanyl-L-leucine éthylamide
le [4-(N-hydroxyamino)-2(RS) -heptylsuccinyl]-L-($N^{\varepsilon}$-phtaloyl) -lysyl-L-leucine éthylamide
le [4-(N-hydroxyamino)-2(RS)-undécylsuccinyl]-L-leucyl-L-leucine éthylamide et
le [4-(N-hydroxyamino)-2(RS)-heptylsuccinyl]-L-phénylalanyl-L-leucine éthylamide.

**24.** Un composé selon la revendication 1 choisi parmi

le [4-(N-hydroxyamino)-2(RS) -heptylsuccinyl]-L-nonalyl-L-leucine éthylamide,
le [4-(N-hydroxyamino)-2 (RS)-heptylsuccinyl]-L-phénylalanine tert-butylamide,
le [4-(N-hydroxyamino)-2(RS)-heptylsuccinyl]-L-tertbutylglycine méthylamide,
le [4-(N-hydroxyamino)-2(RS)-heptylsuccinyl]-L-néopentylglycine méthylamide,
le [4-(N-hydroxyamino)-2(RS) -heptylsuccinyl]-L-homophénylalanyl-L-leucine éthylamide,
le [4-(N-hydroxyamino)-2(RS)-heptylsuccinyl]-L-cyclohexylalanine méthylamide,
le [4-(N-hydroxyamino)-2(RS) -isooctylsuccinyl]-L-phénylalanine méthylamide,
le [4-(N-hydroxyamino)-2(R) -heptylsuccinyl]-L-néopentylglycine méthylamide,
le [4-(N-hydroxyamino)-2(R)-heptylsuccinyl]-(D ou L)-β,β-diméthylphénylalanine méthylamide,
le [4-(N-hydroxyamino) -2(R) -heptylsuccinyl]- (D ou L)-thréo-β-méthylphénylalanine méthylamide,
le [4-(N-hydroxyamino)-2(R)-heptylsuccinyl]-DL-érythro-β-méthylphénylalanine méthylamide et
le [4-(N-hydroxyamino)-2(R)-heptyl-3(R ou S)-[(3-méthyl-2,5-dioxo-1-imidazolidinyl)méthyl]succinyl]-L-leucyl-L-leucine éthylamide.

**25.** Composés selon la formule générale

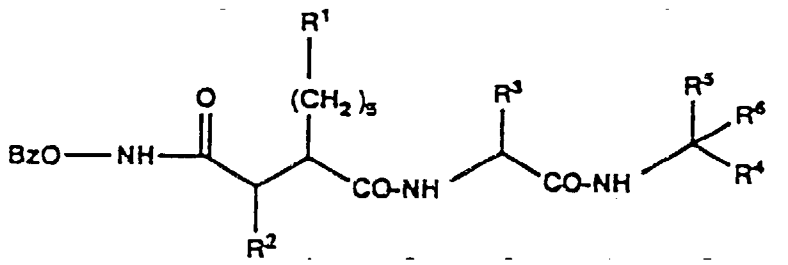

(II)

dans laquelle $R^1$, $R^2$, $R^3$, $R^4$, $R^5$ et $R^6$ ont la signification spécifiée dans la revendication 1 et Bz représente du benzyle.

**26.** Composés de la formule générale :

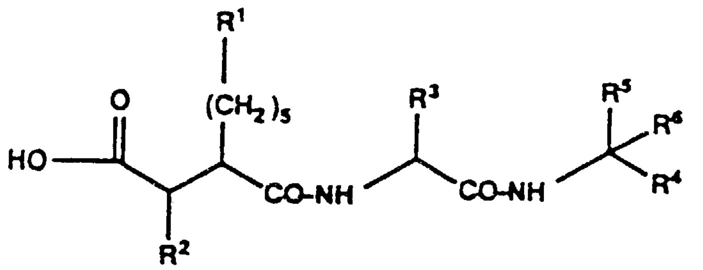

(III)

dans laquelle $R^1$, $R^2$, $R^3$, $R^4$, $R^5$ et $R^6$ ont la signification spécifiée dans la revendication 1, et les esters activés de ceux-ci.

**27.** Composés selon l'une quelconque des revendications 1 à 24, destinés à être utilisés en tant que substances thérapeutiquement actives.

**28.** Composés selon l'une quelconque des revendications 1 à 24, destinés à être utilisés dans le contrôle ou la prévention des maladies dégénératives des articulations ou dans le traitement des tumeurs envahissantes, de l'athérosclérose ou de la sclérose multiple.

**29.** Un procédé pour la fabrication de composés revendiqués dans l'une quelconque des revendications 1 à 24, ce composé comportant :

(a) l'hydrogénation par voie catalytique d'un composé de la formule générale

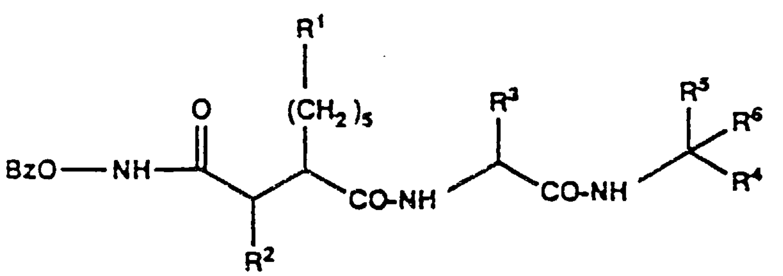

(II)

dans laquelle $R^1$, $R^2$, $R^3$, $R^4$, $R^5$ et $R^6$ ont la signification spécifiée dans la revendication 1 et Bz représente du benzyle, ou bien

(b) la réaction d'un acide de la formule générale

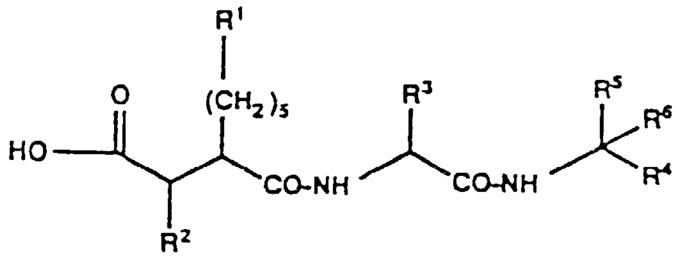

(III)

dans laquelle $R^1$, $R^2$, $R^3$, $R^4$, $R^5$ et $R^6$ ont la signification spécifiée dans la revendication 1, ou bien un ester activé de celui-ci avec un composé de la formule générale

$$H_2N\text{-}OZ \qquad (IV)$$

dans laquelle Z représente de l'hydrogène, un radical tri(alkyl en $C_1$ à $C_6$) silyle ou diphényl (alkyl en $C_1$ à $C_6$) silyle,

et, si cela est exigé, le clivage d'un groupe quelconque diphényl (alkyl en $C_1$ à $C_6$) silyle présent dans le produit de réaction, et,
si on le souhaite, la conversion d'un composé de formule I obtenu en un sel pharmaceutiquement acceptable.

**30.** Un médicament renfermant un composé selon l'une quelconque des revendications 1 à 24 et un matériau support thérapeutiquement inerte.

**31.** Un médicament pour le contrôle ou la prévention des maladies dégénératives des articulations ou pour le traitement des tumeurs envahissantes, de l'athérosclérose ou de la sclérose multiple, qui renferme un composé selon l'une quelconque des revendications 1 à 24 et un matériau support thérapeutiquement inerte.

**32.** L'utilisation d'un composé selon l'une quelconque des revendications 1 à 24 pour la fabrication d'un médicament destiné au contrôle ou à la prévention des maladies dégénératives des articulations ou bien pour le traitement des tumeurs envahissantes, de l'athérosclérose ou de la sclérose multiple.